(19) Europäisches Patentamt
European Patent Office
Office européen des brevets

(11) **EP 4 434 447 A1**

(12) **EUROPEAN PATENT APPLICATION**

(43) Date of publication:
**25.09.2024 Bulletin 2024/39**

(51) International Patent Classification (IPC):
**A61B 5/00** $^{(2006.01)}$

(21) Application number: **24166071.1**

(52) Cooperative Patent Classification (CPC):
**A61B 5/0095; A61B 5/4312;** A61B 2560/0223

(22) Date of filing: **25.03.2024**

| | |
|---|---|
| (84) Designated Contracting States:<br>**AL AT BE BG CH CY CZ DE DK EE ES FI FR GB<br>GR HR HU IE IS IT LI LT LU LV MC ME MK MT NL<br>NO PL PT RO RS SE SI SK SM TR**<br>Designated Extension States:<br>**BA**<br>Designated Validation States:<br>**GE KH MA MD TN**<br><br>(30) Priority: **23.03.2023 US 202363491745 P<br>11.03.2024 US 202418601415** | (71) Applicant: **Seno Medical Instruments, Inc.<br>San Antonio, TX 78230 (US)**<br><br>(72) Inventor: **MO, Larry<br>San Antonio, 78230 (US)**<br><br>(74) Representative: **Leach, Sean Adam et al<br>Mathys & Squire<br>The Shard<br>32 London Bridge Street<br>London SE1 9SG (GB)** |

(54) **METHODS AND SYSTEMS FOR COMPUTING FUNCTIONAL PARAMETERS FOR OPTOACOUSTIC IMAGES**

(57) Optoacoustic (OA) imaging systems and methods are described that obtain OA return signal data associated with a response of a sub-region of a region of interest (ROI) to laser light pulses having one or more predominant wavelengths. An acoustic pressure data set is generated based on the OA return signal data. The acoustic pressure data is dependent on a composition of a first chromophore of interest (COI) and one or more second chromophores not of interest (non-COI) in the sub-region. An extent of the one or more second chromophores within the sub-region is identified. A value is assigned to one or more second chromophore factors based on the extent of the one or more chromophores within the sub-region. An amount of the first chromophore in the sub-region is computed based on the acoustic pressure data and the value assigned to the one or more chromophore factors. The amount of the first chromophore is then utilized to compute parametric maps for display.

FIG. 3

**Description**

CROSS REFERENCE TO RELATED APPLICATIONS

This application claims the benefit of U.S. Provisional Application No. 63/491,745, filed 23-March-2023, the subject matter of which is herein incorporated by reference in its entirety.

BACKGROUND

Worldwide, cancer is a leading cause of death. While numerous types of cancer exist today, some common types of cancer include thyroid cancer, prostate cancer and breast cancer. Breast cancer is the second leading cause of cancer death in women. One of the major challenges for treatment of thyroid, prostate and breast cancer is the heterogeneous nature of each cancer, which determines the therapeutic options. In breast cancer, the somatic genomic landscape of mutations largely influences breast cancer prognosis and therapeutic approach. Breast cancers (as well as other types of cancer) with differing receptor expression and gene amplification profiles have different risk factors for incidence, therapeutic response, disease progression, and preferential organ sites of metastases.

Various imaging modalities are utilized in connection with detection, identification and monitoring progression of different types of cancer. Each type of imaging source yields different information and shows different characteristics of the tissue being imaged. The features apparent in images from one modality differ from the features apparent in images from another modality.

For example, ultrasound is used today in the evaluation of suspicious breast masses, and guiding biopsies. However, ultrasound images present anatomical features in great detail, but do not present functional features associated with cancer in sufficient detail to provide enough prognostic information about cancers, and therefore has limited value to clinicians beyond the assessment of tumor size.

Magnetic resonance imaging (MRI) is utilized for detection of some types of cancer, however MRI is not a primary imaging modality for other types of cancer. For example, MRI imaging of the breast is used as a supplemental tool to breast screening with mammography or ultrasound as the primary modality for imaging. A breast MRI is mainly used for women who have been diagnosed with breast cancer, to help measure the size of the cancer, look for other tumors in the breast, and to check for tumors in the opposite breast. MRI may give some false positive results which mean more test and/or biopsies for the patient (Radhakrishna et al. "Role of magnetic resonance imaging in breast cancer management", South Asian J Cancer 2018;7:69-71). Thus, although breast MRI is useful for women at high risk, it is rarely recommended as a screening test for women at average risk of breast cancer (Radhakrishna et al.). Also, breast MRI does not show calcium deposits, known as micro-calcifications which can be a sign of breast cancer (Radhakrishna et al.).

An entirely different modality has been proposed more recently in connection with imaging, namely optoacoustic (OA) imaging. OA imaging uses pulsed laser light to illuminate biological tissue. When the incident light energy is absorbed by tissue molecules known as chromophores, they undergo transient thermoelastic expansion which gives rise to acoustic waves that can be detected by an array of ultrasound transducers. The received array signals can be processed to create an image of the chromophore spatial distribution to within the resolution determined by the transducer array characteristics and provides great detail regarding functional features of the region of interest.

OA imaging is an emerging imaging modality with both clinical and preclinical applications, as it is capable of generating functional information that complements the anatomical information from conventional gray-scale ultrasound imaging. Specifically, in OA imaging, endogenous hemoglobin acts as a dominating chromophore allowing imaging of total hemoglobin concentration and relative hemoglobin oxygen saturation. For preclinical applications, exogenous contrast agents including nanoparticles may be externally administered to enable molecular imaging. For both preclinical and clinical studies, image generation in an OA system includes 3 stages: I) OA sinogram acquisition, II) OA image reconstruction, and III) post-processing including functional parameter estimation & display.

In stage I, a laser pulse is transmitted to illuminate tissue at a first wavelength $\lambda 1$. The light pulse propagates through the tissue medium but is attenuated and scattered. Chromophores in the tissue medium absorb light energy, heat up, and generate acoustic waves that travel through the tissue and are detected as radio frequency (RF) signals at an ultrasound detection array. The receive system performs RF signal amplification and digitization to form a sinogram, which refers to a 2D matrix of time series raw OA data collected for each channel of a transducer array.

In stage II, the system digitally filters the sinogram and performs OA image reconstruction to produce an OA image. The OA image includes OA pressure estimates $p_o(\lambda 1, x)$ for each pixel location $x=(x,y,z)$ in the cartesian coordinate system. The above process is repeated at a second wavelength $\lambda 2$ to generate a second OA image that includes OA pressure estimates $p_o(\lambda 2, x)$ based on the second wavelength $\lambda 2$. In the reconstructed $p_o(\lambda i, x)$ images, each pixel can be interpreted as the sum of contributions from a mixture of chromophore types, where the chromophore types are weighted and the weights represent the average concentration of the corresponding chromophore.

The first two stages of OA image generation are dependent on the ultrasound detector array size and geometry. Pre-clinical OA systems often leverage circular or semi-circular transducer arrays whose field of view covers an entire cross-sectional area of small animals, enabling tomographic reconstruction of the OA source pressure field. Like x-ray Computed Tomography (CT), the OA reconstruction problem can be formulated mathematically as an inverse problem, and various iterative algorithms may utilize convergence criteria that leverage prior knowledge, penalties on outliers, and known constraints. In contrast, for clinical OA or hybrid OA-US systems, the US transducer array is not large enough to scan an entire field of view while remaining stationary. Hence, reconstructing entire functional and anatomical features within the field of view is very challenging due to the limited view angles relative to the anatomy being imaged.

The stage III post-processing includes fluence normalization which adjusts the OA pressure estimate $p_o(\lambda 1, x)$ for each pixel to compensate for wavelength-dependent optical attenuation and scattering, as a function of depth, experienced by the received OA signals. The attenuation and scattering affect the amplitude of the received OA signals in connection with each point (voxel) in the region of interest. Fluence normalization generally utilizes standard optical attenuation and scattering models without regard for the nature of an individual patient.

Stage III post-processing also includes a source unmixing computation, which is also commonly known as spectral unmixing. Source unmixing seeks to resolve how much each of two or more chromophores may have contributed to the total observed optical absorption at different spectral wavelengths. The estimated chromophore concentrations are then converted to functional information including the total hemoglobin concentration and oxygen saturation level. The functional parametric maps are displayed, such as an overlay to the background anatomical US image.

Like OA image reconstruction, the specific challenges in OA post-processing are also quite different between preclinical and clinical systems. In the former case, a small animal is usually anesthetized, so that multiple (>5) laser firings that span a wide range of wavelengths can be used to measure the optical absorption as a function of wavelength, without concerns about motion artifacts. This is referred to as multispectral optoacoustic tomography (MSOT), which may utilize a custom-designed, large US array, or a clinical US transducer array. In addition, exogenous contrast agents can be externally administered for different molecular imaging applications. Given a relatively large number of $p_0$ measurements at different wavelengths, a variety of blind and model-based MSOT unmixing techniques have been proposed.

However, the MSOT unmixing techniques are not well suited for clinical applications where the anatomy of interest is larger than the transducer array field of view and where the patient is not anesthetized. For clinical applications, the OA acquisition parameters and targets are quite different. State-of-the-art clinical hybrid OA-US platforms generally involve integrating a laser system to a clinical ultrasound system and array transducers for free-hand scanning of the anatomy of interest. Due to both cost and acquisition time considerations, the number of laser firings for each image frame must be relatively small. In a preferred embodiment, only two laser firings at different wavelengths are used to measure the relative concentrations of oxygenated and deoxygenated hemoglobin concentrations. Exogenous contrast agents are not used as they are still in the research phase. Because of these differences, the preclinical MSOT unmixing methods are generally not suited for clinical OA applications.

The foregoing problems are discussed in the context of using OA imaging to analyze hemoglobin related characteristics of OA images for breast cancer. However, OA imaging can also be applied to analyze other characteristics, such as for thyroid cancer, prostate cancer and breast cancer.

Further OA imaging may be utilized to analyze patient tissue for non-cancerous characteristics. For example, new interest has arisen regarding the use of OA imaging to analyze kidney fibrosis (e.g., in connection with determining viability of candidate transplant kidneys). Fibrosis is a form of chronic damage that may be present in donor kidneys, and it is an important predictor of future renal function. It has been proposed to potentially utilize OA imaging techniques to directly images collagen, the principal component of fibrotic tissue. OA imaging can be utilized to noninvasively quantify whole kidney fibrotic burden with sufficiently high resolution to capture intrarenal variations in collagen content.

A need remains for improvements in stage III post-processing for clinical OA imaging.

SUMMARY

In an aspect there is provided an optoacoustic imaging system as defined in claim 1. In another aspect there is provided an optoacoustic imaging system as defined in claim 11. Embodiments are set out in the dependent claims.

In accordance with embodiments herein, an optoacoustic (OA) imaging system is provided that comprises one or more light sources configured to generate a first laser light pulse having a first predominant wavelength; an OA probe operatively coupled to the one or more light sources, the OA probe configured to deliver the first laser light pulse to a region of interest (ROI) of tissue, the ROI including i) at least one non-hemoglobin (non-Hb) chromophore, and ii) at least one hemoglobin (Hb) chromophore, the OA probe including a transducer array that is configured to collect OA return signal data associated with a response of the ROI to one or more of the first laser light pulses; memory configured to store program instructions; and one or more processors configured to execute the programmable instructions to: generate an acoustic pressure data set based on the OA return signal data, the acoustic pressure data associated with the sub-region of the ROI, wherein the acoustic pressure data is dependent on a composition of hemoglobin (Hb) and non-Hb chromophores in the sub-region; identify an extent of the at least one non-Hb chromophore within the sub-region; assign a value to a non-Hb chromophore factor based on the extent of the at least one non-Hb chromophore within the sub-region; and compute an amount of at least one of i) the Hb chromophore or ii) a second non-Hb chromophore in the sub-region based on the acoustic pressure data and the value assigned to the non-Hb chromophore factor.

Additionally or alternatively, the one or more processors are further configured to identify the extent of the at least one non-Hb chromophore within the sub-region, by at least one of: i) analyzing an imaging data set for the sub-region to determine an aspect of the composition related to the extent of the one non-Hb chromophore; or ii) analyzing pathology data indicative of the extent of the non-Hb chromophore; or iii) receiving patient data indicative of the extent of the non-Hb chromophore.

Additionally or alternatively, the ROI includes first and second sub-regions having different first and second compositions, the one or more processors further configured to identify first and second extents of the non-Hb chromophore within the first and second sub-regions, respectively, and, based thereon, to assign first and second values to the non-Hb chromophore factor for the first and second sub-regions, respectively.

Additionally or alternatively, the one or more processors are further configured to repeat the generate, identify, assign and compute operations for at least one of: i) multiple sub-regions throughout the ROI, ii) multiple positions throughout the subregion, or iii) multiple positions throughout multiple sub-regions throughout the ROI.

Additionally or alternatively, to identify the extent, the one or more processors are further configured to, at least one of: i) determine a volume fraction of the non-Hb chromophore in the sub-region, ii) classify the sub-region into one or more tissue types, or iii) determine a concentration of the non-Hb chromophore in the sub-region.

Additionally or alternatively, the acoustic pressure data for the ROI represents an OA image and wherein the one or more processors are further configured to: apply a fluence adjustment to the acoustic pressure data of the OA image to form a fluence-adjusted OA image; and compute a parametric map based on the fluence-adjusted OA image after applying the fluence adjustment.

Additionally or alternatively, the one or more processors are further configured to assign the value for the non-Hb chromophore factor based on at least one of: i) an optical absorption coefficient of the non-Hb chromophore in the sub-region, ii) an optical extinction coefficient and molar concentration of the non-Hb chromophore in the sub-region, iii) a mass concentration and molecular weight of the non-Hb chromophore in the sub-region, or iv) a volume fraction of the non-Hb chromophore in the sub-region.

Additionally or alternatively, the non-Hb chromophore factor corresponds to at least one of: i) an optical absorption coefficient of the non-Hb chromophore in the sub-region, ii) an optical extinction coefficient of the non-Hb chromophore in the sub-region, iii) a molar concentration of the non-Hb chromophore in the sub-region, iv) a mass concentration of the non-Hb chromophore in the sub-region, or iv) a volume fraction of the non-Hb chromophore in the sub-region.

Additionally or alternatively, the non-Hb chromophore factor corresponds to a non-blood absorption coefficient and wherein, to compute the amount of the Hb chromophore, the one or more processors are further configured to: determine a tissue absorption coefficient based on the acoustic pressure data; determine a blood absorption coefficient based on the tissue absorption coefficient and the value of the non-blood absorption coefficient; and determine the amount of the Hb chromophore based on the blood absorption coefficient and an Hb extinction coefficient.

Additionally or alternatively, the one or more processors are further configured to compute a parametric map based on the amount of the Hb chromophore.

Additionally or alternatively, the acoustic pressure data includes a collection of pressure data values representative of an acoustic response at corresponding positions throughout the sub-region of the ROI.

Additionally or alternatively, the amount of the Hb chromophore includes a first amount for an oxygenated Hb (HbO) chromophore and a second amount for a de-oxygenated Hb (HbR) chromophore.

Additionally or alternatively, the non-Hb chromophore includes at least one of a water chromophore, melanin chromophore or lipid chromophore, or wherein the Hb chromophore includes at least one of an oxygenated hemoglobin (HbO) chromophore or a deoxygenated hemoglobin (HbR) chromophore.

In accordance with embodiments herein, an OA system is provided that comprises: memory configured to store program instructions and to store OA return signal data associated with a response of a sub-region of a region of interest (ROI) to one or more first laser light pulses having a first predominant wavelength; and one or more processors configured to execute the programmable instructions to: obtain OA return signal data associated with a response of a sub-region of a region of interest (ROI) to one or more first laser light pulses having a first predominant wavelength; generate an acoustic pressure data set based on the OA return signal data, the acoustic pressure data associated with the sub-region of the ROI, wherein the acoustic pressure data is dependent on a composition of hemoglobin (Hb) and non-Hb chromophores in the sub-region; identify an extent of the non-Hb chromophore within the sub-region; assign a value to a non-Hb chromophore factor based on the extent of the non-Hb chromophore within the sub-region; and compute an amount of the Hb chromophore in the sub-region based on the acoustic pressure data and the value assigned to the non-Hb chromophore factor.

Additionally or alternatively, the system further comprises a network service housing the memory and the one or more processors, the network service configured to receive, over a network connection, at least one of the OA return signal data or acoustic pressure data from one or more OA imaging systems.

Additionally or alternatively, the system further comprises an OA imaging system that includes one or more light sources configured to generate a first laser light pulse having a first predominant wavelength; and an OA probe operatively coupled to the one or more light sources, the OA probe configured to deliver the first laser light pulse to a region of interest (ROI) of tissue, the ROI including i) at least one non-hemoglobin (non-Hb) type chromophore, and ii) at least one Hb type chromophore, the OA probe including a transducer array that is configured to collect the OA return signal data associated with a response of the ROI to one or more of the first laser light pulses.

The optoacoustic imaging system described and claimed herein may comprise one or more light sources configured to generate a first laser light pulse having a first predominant wavelength; an OA probe operatively coupled to the one or more light sources, the OA probe configured to deliver the first laser light pulse to a region of interest (ROI) of tissue, the ROI including i) at least one non-hemoglobin (non-Hb) chromophore, and ii) at least one hemoglobin (Hb) chromophore, and the OA probe including a transducer array that is configured to collect OA return signal data associated with a response of the ROI to one or more of the first laser light pulses. Accordingly, the one or more processors may thus obtain the OA return signal data associated with a response of a sub-region of the ROI to the one or more first laser light pulses having the first predominant wavelength.

In accordance with embodiments herein an OA imaging method is provided that comprises: delivering a first laser light pulse, having a first predominant wavelength, a region of interest (ROI) of tissue, the ROI including i) at least one non-hemoglobin (non-Hb) chromophore, and ii) at least one hemoglobin (Hb) chromophore, collecting OA return signal data associated with a response of the ROI to one or more of the first laser light pulses; utilizing one or more processors configured to execute the programmable instructions for: generating an acoustic pressure data set based on the OA return signal data, the acoustic pressure data associated with the sub-region of the ROI, wherein the acoustic pressure data is dependent on a composition of hemoglobin (Hb) and non-Hb chromophores in the sub-region; identifying an extent of the at least one non-Hb chromophore within the sub-region; assigning a value to a non-Hb chromophore factor based on the extent of the at least one non-Hb chromophore within the sub-region; and computing an amount of at least one of i) the Hb chromophore or ii) a second non-Hb chromophore in the sub-region based on the acoustic pressure data and the value assigned to the non-Hb chromophore factor.

Additionally or alternatively, the identifying the extent of the at least one non-Hb chromophore within the sub-region, includes at least one: i) analyzing an imaging data set for the sub-region to determine an aspect of the composition related to the extent of the one non-Hb chromophore; or ii) analyzing pathology data indicative of the extent of the non-Hb chromophore; or iii) receiving patient data indicative of the extent of the non-Hb chromophore.

Additionally or alternatively, the ROI includes first and second sub-regions having different first and second compositions, the method comprising identifying first and second extents of the non-Hb chromophore within the first and second sub-regions, respectively, and, based thereon, assigning first and second values to the non-Hb chromophore factor for the first and second sub-regions, respectively.

Additionally or alternatively, the method further comprises repeating the generating, identifying, assigning and computing operations for at least one of: i) multiple sub-regions throughout the ROI, ii) multiple positions throughout the subregion, or iii) multiple positions throughout multiple sub-regions throughout the ROI.

Additionally or alternatively, the identifying the extent, includes at least one of: i) determining a volume fraction of the non-Hb chromophore in the sub-region, ii) classifying the sub-region into one or more tissue types, or iii) determining a concentration of the non-Hb chromophore in the sub-region.

Additionally or alternatively, the acoustic pressure data for the ROI represents an OA image and wherein the method further comprises: applying a fluence adjustment to the acoustic pressure data of the OA image to form a fluence-adjusted OA image; and computing a parametric map based on the fluence-adjusted OA image after applying the fluence adjustment.

Additionally or alternatively, the method further comprises assigning the value for the non-Hb chromophore factor based on at least one of: i) an optical absorption coefficient of the non-Hb chromophore in the sub-region, ii) an optical extinction coefficient and molar concentration of the non-Hb chromophore in the sub-region, iii) a mass concentration and molecular weight of the non-Hb chromophore in the sub-region, or iv) a volume fraction of the non-Hb chromophore in the sub-region.

Additionally or alternatively, the non-Hb chromophore factor corresponds to at least one of: i) an optical absorption coefficient of the non-Hb chromophore in the sub-region, ii) an optical extinction coefficient of the non-Hb chromophore in the sub-region, iii) a molar concentration of the non-Hb chromophore in the sub-region, iv) a mass concentration of the non-Hb chromophore in the sub-region, or iv) a volume fraction of the non-Hb chromophore in the sub-region.

Additionally or alternatively, the non-Hb chromophore factor corresponds to a non-blood absorption coefficient and wherein, the computing the amount of the Hb chromophore includes: determining a tissue absorption coefficient based on the acoustic pressure data; determining a blood absorption coefficient based on the tissue absorption coefficient and the value of the non-blood absorption coefficient; and determining the amount of the Hb chromophore based on the blood absorption coefficient and an Hb extinction coefficient.

Additionally or alternatively, the method further comprises computing a parametric map based on the amount of the Hb chromophore.

Additionally or alternatively, the acoustic pressure data includes a collection of pressure data values representative of an acoustic response at corresponding positions throughout the sub-region of the ROI.

Additionally or alternatively, the amount of the Hb chromophore includes a first amount for an oxygenated Hb (HbO) chromophore and a second amount for a de-oxygenated Hb (HbR) chromophore.

Additionally or alternatively, the non-Hb chromophore including at least one of a water chromophore, melanin chromophore or lipid chromophore, or wherein the Hb chromophore includes at least one of an oxygenated hemoglobin (HbO) chromophore or a deoxygenated hemoglobin (HbR) chromophore.

The optoacoustic imaging methods described and claimed herein may comprise delivering a first laser light pulse, having a first predominant wavelength, a region of interest (ROI) of tissue, the ROI including i) at least one non-hemoglobin (non-Hb) chromophore, and ii) at least one hemoglobin (Hb) chromophore, and collecting OA return signal data associated with a response of the ROI to one or more of the first laser light pulses; thereby to obtain the OA return signal data associated with a response of the ROI to the one or more of first laser light pulses.

In accordance with embodiments herein, an OA method is provided that comprises: obtaining OA return signal data associated with a response of a sub-region of a region of interest (ROI) to one or more first laser light pulses having a first predominant wavelength; utilizing one or more processors configured to execute the programmable instructions for: generating an acoustic pressure data set based on the OA return signal data, the acoustic pressure data associated with the sub-region of the ROI, wherein the acoustic pressure data is dependent on a composition of a first chromophore and one or more second chromophores in the sub-region; identifying an extent of the one or more second chromophores within the sub-region; assigning a value to one or more second chromophore factors based on the extent of the one or more chromophores within the sub-region; and computing an amount of the first chromophore in the sub-region based on the acoustic pressure data and the value assigned to the one or more chromophore factors.

Additionally or alternatively, the method further comprises receiving, at a network service housing the memory and the one or more processors, at least one of the OA return signal data or acoustic pressure data from one or more OA imaging systems.

Additionally or alternatively, the first chromophore represents a hemoglobin (Hb) chromophore and the one or more second chromophores represent one or more non-Hb chromophores.

Additionally or alternatively, the first chromophore represents a collagen chromophore and the one or more second chromophores represent one or more non-collagen chromophores.

Additionally or alternatively, the first chromophore represents a lipid chromophore and the one or more second chromophores represent one or more non-lipid chromophores.

BRIEF DESCRIPTION OF THE DRAWINGS

Figure 1 illustrates a computing subsystem for computing Hb chromophore content in accordance with embodiments herein.

Figure 2 plots the optical absorption coefficient vs wavelength for the main chromophores in a patient's ROI that have a diagnostically relevant impact on results of OA imaging.

Figure 3 illustrates a collection of modules implemented by the computing subsystem in more detail, and in particular, the chromophore unmixing sub-system implemented in accordance with embodiments herein.

Figure 4 illustrates a block diagram of an OA imaging system that implements the computing subsystem of Figure 3 in accordance with embodiments herein.

Figure 5A illustrates a method for determining chromophore factor values in accordance with embodiments herein.

Figure 5B illustrates a method for determining chromophore factor values in accordance with alternative embodiments herein.

Figure 6 shows a schematic block diagram illustrating an embodiment of a combined optoacoustic and ultrasound system that may be used as a platform for the methods and devices disclosed herein.

Figure 7 illustrates an overview of a collection of operations for processing OA/US data.

Figure 8 is a block diagram illustrating a web-based US/OA image management system formed in accordance with embodiments herein.

DETAILED DESCRIPTION

The following description and drawings are illustrative and are not to be construed as limiting. Numerous specific details are described to provide a thorough understanding. However, in certain instances, well-known or conventional details are not described in order to avoid obscuring the description. References to one or an embodiment in the present disclosure are not necessarily references to the same embodiment; and such references mean at least one.

Reference in this specification to "one embodiment" or "an embodiment" means that a particular feature, structure, or characteristic described in connection with the embodiment is included in at least one embodiment of the disclosure. The appearances of the phrase "in one embodiment" in various places in the specification are not necessarily all referring to the same embodiment, nor are separate or alternative embodiments mutually exclusive of other embodiments. Moreover, various features are described which may be exhibited by some embodiments and not by others. Similarly, various requirements are described which may be requirements for some embodiments, but not other embodiments.

The systems and methods are described below with reference to, among other things, block diagrams, operational illustrations and algorithms of methods and devices to provide optoacoustic imaging with out-of-plane artifact suppression. It is understood that each block of the block diagrams, operational illustrations and algorithms and combinations of blocks in the block diagrams, operational illustrations and algorithms, can be implemented by means of analog or digital hardware and computer program instructions.

Reference will now be made in more detail to various embodiments of the present invention, examples of which are illustrated in the accompanying figures. As will be apparent to one of skill in the art, the data structures and processing steps described herein may be implemented in a variety of other ways without departing from the spirit of the disclosure and scope of the invention herein and should not be construed as being limited to the embodiments set forth herein. Rather, these embodiments are provided so that this disclosure will be thorough and complete, and will fully convey the concept of the disclosure to those skilled in the art.

In accordance with embodiments herein, methods and systems are described in one or more attachments to this specification where the related description is set forth in outline format, summary format, images and the like. All the material submitted with and/or attached to the present specification is expressly incorporated herein by reference in its entirety.

Overview

Embodiments herein describe new and unique methods and systems for post-processing of acoustic pressure data sets collected by OA imaging systems that use laser light pulses with a relatively small number of optical (laser) wavelengths. Embodiments herein can be integrated into a multi-wavelength (e.g., 2 wavelength) hybrid OA-US system, such as described in US Patent 9,289,191B2, titled "System and Method for Acquiring Optoacoustic Data and Producing Parametric Maps Thereof', the complete subject matter of which is expressly incorporated herein by reference in its entirety. The system of the '191 patent acquires one or more sets of OA data associated with a first optical wavelength $\lambda_1$ and one or more sets of OA data associated with a second optical wavelength $\lambda_2$. The raw OA data is also referred to as OA return signal data. The system processes the OA return signal data to generate a corresponding acoustic pressure data set. The acoustic pressure data set may also be referred to as an OA image, however, as noted in the '191 patent the OA "image" is not necessarily a visual representation of the underlying acoustic pressure data. The system applies spatial smoothing and fluence normalization to the acoustic pressure data (e.g., OA image), followed by an "unmixing" operation.

The unmixing operation is a process by which the acoustic pressure data value(s) for a specific point or sub-region in the ROI (e.g., pixel or pixels, voxel or voxels or other location specific data points) are decomposed into a collection of constituent chromophores of interest, such as to separate HbO, HbR, and non-Hb types of chromophores. Additionally or alternatively, the unmixing operation may decompose the acoustic pressure data values to separate the collagen chromophore from non-collagen chromophores, the lipid chromophore from non-lipid chromophores and the like. A set of corresponding fractions or values for chromophore factors are then be utilized to model or otherwise indicate the proportion of each member (chromophore) that caused or produced the acoustic pressure data for a corresponding specific data point or sub-region in the ROI. More specifically, acoustic pressure data for each spatial location is unmixed thereby providing estimates of $c_{Hb0}$ and $c_{HbR}$, representing the mass concentrations of oxygenated and deoxygenated hemoglobin in blood respectively. The $c_{Hb0}$ and $c_{HbR}$ estimates, in turn, may be used for various purposes such as to compute functional parametric maps of the total blood hemoglobin concentration ($c_{tHb}$) and oxygenation level ($SO_2$). In embodiments herein, an ultrasound (US) image may also be acquired, and the parametric map co-registered with and overlaid upon the ultrasound image, and then displayed. The $c_{tHb}$ and $SO_2$ parametric images can respectively highlight areas where the total hemoglobin concentration is high, and where the blood oxygenation level is low, relative to tissue baseline values.

If it is assumed that the illuminated tissue contains solely oxygenated and deoxygenated hemoglobin molecules, then by making OA signal measurements in response to first and second laser light pulses having corresponding dominant wavelengths $\lambda_1$ and $\lambda_2$, a forward model can be described by a 2x2 matrix equation, such that $c_{Hb0}$ and $c_{HbR}$ can be computed by taking the inverse of a 2x2 matrix. However, in reality, there are other chromophores in tissue that can contribute to the received OA signals. In the optical wavelength range suitable for clinical OA, one substantial non-Hb chromophore is water, while others include lipid and melanin.

In accordance with new and unique aspects herein, methods and systems are described to account for the contributions from the non-Hb chromophores (e.g., water, lipid, melanin). For example, embodiments herein incorporate, into forward models, fractional volumes of the primary non-Hb chromophores in the tissue being illuminated. In accordance with new and unique aspects herein, the methods and systems herein can be applied to improve stage III post-processing to improve the specificity and sensitivity of the OA imaging system with respect to one or more chromophores of interest (e.g., oxygenated and deoxygenated hemoglobin, collagen, lipid, water, etc.) within sub-regions of an ROI. By improving the specificity and sensitivity of the OA imaging system with respect to one or more chromophores of interest in OA images, methods and systems herein enable better assessment of anatomical and functional behavior/condition of the tissue in the ROI, which in turn improves treatment and prophylaxis (e.g., actions taken to treat/prevent disease) disease. For example, by improving the specificity and sensitivity of the OA imaging system with respect to Hb chromophores, the OA images enable clinicians to better diagnose malignant v. benign tumors, to better diagnosis molecular sub-type and/or histologic grade of a tumor, to better assess kidney fibrosis burden (e.g., which in turn improves assessment of transplant candidate kidneys), and the like.

Conventional fluence normalization has been realized using a model-based approach that uses the average literature values of the tissue optical parameters including $\mu_a(\lambda)$ and $\mu_s(\lambda)$. However, in accordance with new and unique aspects herein, it has been recognized that patient-to-patient variations can lead to significant biases in the resultant functional parameter estimates when such average literature values are used for all patients. Further compounding this problem is that clinical OA waveforms generated in biological tissue often exhibit a random interference pattern known as speckle. Together the estimation biases and variances create a potential to limit the sensitivity and specificity of OA images for cancerous tissue detection and/or characterization. By affording a mechanism to identify an extent of non-Hb chromophores within an ROI and more specifically within sub-regions of the ROI, and to assign corresponding values to non-Hb chromophore factors, embodiments herein significantly improve the accuracy and reliability of the HbT and $SO_2$ estimates. The identification of the extent of chromophores not of interest (e.g., the non-Hb chromophores or non-collagen chromophores) and assignment of a corresponding value to the chromophores not of interest can be adaptively based on various factors such as tissue subtype of each individual patient. For example, to identify the extent of the at least one chromophore not of interest, the methods and systems may perform at least one of: i) analyzing an imaging data set for the sub-region to determine an aspect of the composition related to the extent of the one non-Hb chromophore; or ii) analyzing pathology data indicative of the extent of the non-Hb chromophore; or iii) receiving patient data indicative of the extent of the non-Hb chromophore.

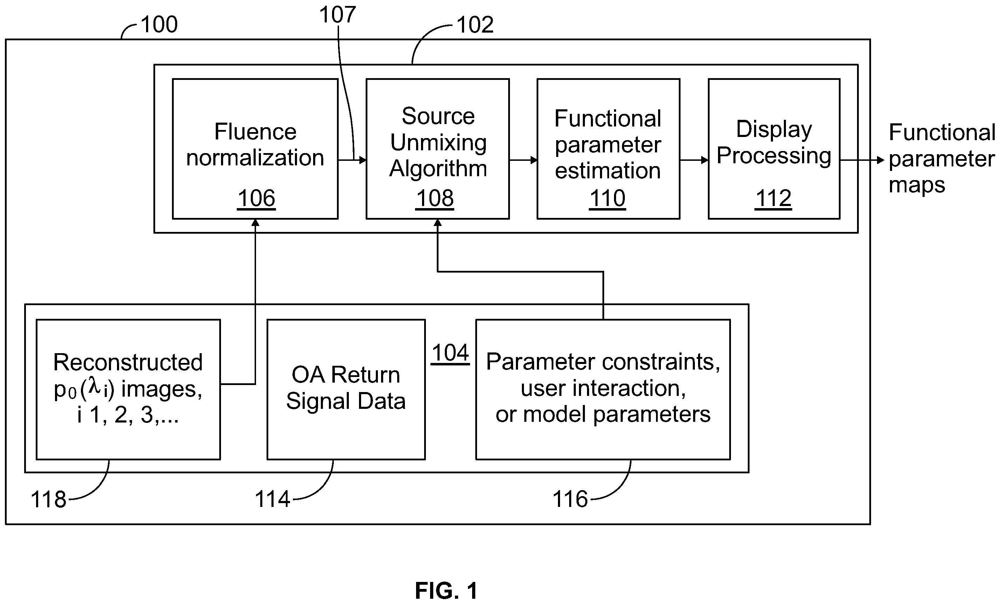

Figure 1 illustrates a computing subsystem 100 for computing a content of a chromophore of interest (e.g., Hb chromophore content, collagen chromophore content, etc.) in accordance with embodiments herein. The computing subsystem 100 includes memory 104 configured to store program instructions and includes one or more processors 102 configured to execute the programmable instructions to perform the operations described herein in connection computing, among other things, an amount of the Hb chromophore in an ROI, and more specifically in subregions of the ROI. The computing subsystem 100 receives and stores, in the memory 104, OA return signal data 114. The OA return signal data 114 is collected by an OA imaging system, as described herein and/or as described in one or more of the patents and publications referenced herein. The OA imaging system includes one or more light sources configured to generate a first laser light pulse having a first predominant wavelength. An OA probe is operatively coupled to the one or more light sources. The OA probe is configured to deliver the first laser light pulse to an ROI of tissue. The ROI includes i) at least one non-hemoglobin (non-Hb) type chromophore, and ii) at least one Hb type chromophore. The OA probe includes a transducer array that is configured to collect the OA return signal data associated with a response of the ROI to one or more of the first laser light pulses.

While embodiments herein describe the use of an OA probe with first and second predominant wavelengths, it is recognized the fewer or more predominant wavelengths may be used based on the type of tissue being images. For example, a third laser light pulse having a third predominant wavelength may be utilized to generate an acoustic pressure data set indicative of collagen. The third laser light pulse may be used alone or in combination with the first and second laser light sources and/or in combination with yet laser light sources having other predominant wavelengths.

The one or more processors 102 of the computing subsystem 100 generate an acoustic pressure data set 118 based on the OA return signal data 114. The acoustic pressure data 118 may be stored in the memory 104 or elsewhere with a network. The acoustic pressure data 118 is associated with a corresponding sub-region of the ROI, wherein the acoustic pressure data 118 is dependent on a composition of Hb chromophores and non-Hb chromophores in the corresponding sub-region. In accordance with embodiments herein, the one or more processors 102 may generate an OA image (as denoted at 118 as "reconstructed images") from the OA return signal data 114, in which case the acoustic pressure data 118 for the ROI includes and/or represents the OA image.

The memory 104 may further store parameter constraints, user interaction inputs, and/or model parameters 116 that are supplied to, and utilized by, the source unmixing algorithm 108. For example, the user interaction may include patient data, pathology data, and the like.

At module 106, the computing subsystem 100 applies a fluence adjustment to the acoustic pressure data 118 of the OA image to form fluence-adjusted acoustic pressure data 107, and in some instances a fluence-adjusted OA image. For example, the fluence adjustment may include fluence normalization which adjusts the acoustic pressure data $p_o(\lambda 1, x)$ for each location in the ROI (e.g., each pixel, voxel or other subregion in an OA image) to compensate for wavelength-dependent optical attenuation and scattering, as a function of depth, exhibited in the received OA return signal data. Optionally, the fluence normalization may be calculated utilizing a predefined value for the optical absorption coefficient $\mu_a(\lambda)$ for an ROI and a predefined value for the optical scattering coefficient $\mu_s(\lambda)$ for the ROI. In accordance with new and unique aspects herein, embodiments may calculate the fluence normalization utilizing separate values for the optical absorption coefficient $\mu_a(\lambda)$ and optical scattering coefficient $\mu_s(\lambda)$ for each sub-region of the ROI, where the optical absorption coefficient $\mu_a(\lambda)$ and optical scattering coefficient $\mu_s(\lambda)$ values are determined based on the particular patient. For example, the optical absorption coefficient $\mu_a(\lambda)$ and optical scattering coefficient $\mu_s(\lambda)$ values may be determined based on an extent of one or more non-Hb chromophores within each corresponding sub-region of the particular patient.

At module 108, the computing subsystem 100 performs an unmixing operation by which the acoustic pressure data value for multiple individual specific locations in the ROI (e.g., pixel, voxel or other location specific data point) is decomposed into a collection of constituent chromophores of interest, namely separate HbO, HbR, and non-Hb chromophores. In general, biological tissue can be modeled as a mixture of several different chromophores including melanin, hemoglobin, water and lipids. Melanin is present mainly in the epidermal layer. Hemoglobin (Hb), the main molecule responsible for oxygen transport in red blood cells, is a special chromophore in that the optical absorption coefficient of Hb varies significantly with its oxygenation state. Hence, for OA imaging, the Hb molecules in blood are further divided into the oxygenated chromophore subgroup (HbO chromophore), and the deoxygenated subgroup (HbR chromophore).

At 108, the one or more processors 102 further identify an extent of the non-Hb chromophore within each of one or more sub-regions and assign a value to a non-Hb chromophore factor based on the extent of the non-Hb chromophore within the corresponding one or more sub-regions. Additionally or alternatively, at 108, the one or more processors 102 may identify an extent of multiple individual non-Hb chromophores within each of one or more sub-regions and assign separate corresponding values to separate corresponding non-Hb chromophore factors based on the extent of the corresponding non-Hb chromophores within the one or more sub-regions. At 108, the one or more processors 102 further compute an amount of the Hb chromophore in each of the one or more sub-regions based on the acoustic pressure data and the value(s) assigned to the non-Hb chromophore factor(s). The ROI may include first and second sub-regions having different first and second compositions. The one or more processors 102 is configured to identify first and second extents of the non-Hb chromophore within the first and second sub-regions, respectively, and, based thereon, to assign first and second values to the non-Hb chromophore factor for the first and second sub-regions, respectively. For example, the first sub-region may include a substantial amount of fatty tissue, while the second sub-region may include a substantial amount of blood. As another example, the first sub-region may include a majority of water or lipids while the second sub-region includes a majority of melanin. It is recognized that an ROI may be divided into a large number of sub-regions, in which case, the one or more processors are further configured to repeat the generate, identify, assign and compute operations for at least one of: i) multiple sub-regions throughout the ROI, ii) multiple positions throughout the subregion, or iii) multiple positions throughout multiple sub-regions throughout the ROI. Each sub-region may correspond to one or a few pixels or voxels, in which case a large number of sub-regions may be primarily composed of blood, while some sub-regions are composed of non-fatty tissue, and other sub-regions are composed of fatty tissue, water, lipids, melanin and/or a combination thereof.

In accordance with embodiments herein, for each sub-region, the one or more processors 102 are configured to, at least one of, determine a volume fraction of the non-Hb chromophore in the sub-region, classify the sub-region into one or more composition types (e.g., tissue, fatty tissue, blood, water, lipid dominated, collagen, melanin dominated), and/or determine a concentration of the non-Hb chromophore in the sub-region. The one or more processors 102 are further configured to identify the extent of the non-Hb chromophore within the sub-region, by at least one of: i) analyzing an ultrasound data set for the sub-region to determine an aspect of the composition related to the extent of the non-Hb chromophore; or ii) receiving patient data indicative of the extent of the non-Hb chromophore.

In accordance with embodiments herein, the one or more processors 102 are configured to determine the value for the non-Hb chromophore factor based on at least one of: i) an optical absorption coefficient of the non-Hb chromophore in the sub-region, ii) an optical extinction coefficient and molar concentration of the non-Hb chromophore in the sub-region, iii) a mass concentration and molecular weight of the non-Hb chromophore in the sub-region, or iv) a fractional volume of the non-Hb chromophore in the sub-region. The non-Hb chromophore factor may correspond to at least one of: i) an optical absorption coefficient of the non-Hb chromophore in the sub-region, ii) an optical extinction coefficient of the non-Hb chromophore in the sub-region, iii) a molar concentration of the non-Hb chromophore in the sub-region, iv) a mass concentration of the non-Hb chromophore in the sub-region, or iv) a fractional volume of the non-Hb chromophore in the sub-region.

For example, the non-Hb chromophore factor may correspond to a non-blood absorption coefficient. To compute the amount of the Hb chromophore, the one or more processors 102 are further configured to: determine a tissue absorption coefficient based on the acoustic pressure data; determine a blood absorption coefficient based on the tissue absorption coefficient and the value of the non-blood absorption coefficient; and determine the amount of the Hb chromophore based on the blood absorption coefficient and an Hb extinction coefficient.

At 110, the one or more processors 102 compute a parametric map based on the amount of the Hb chromophore. The acoustic pressure data may include a collection of pressure data values representative of an acoustic response at corresponding positions throughout the sub-region of the ROI. Additionally or alternatively, the amount of the Hb chromophore may include a first amount for an oxygenated Hb (HbO) chromophore and a second amount for a de-oxygenated Hb (HbR) chromophore. Additionally or alternatively, the non-Hb chromophore may include at least one of a water chromophore, melanin chromophore or lipid chromophore, or wherein the Hb chromophore includes at least one of an oxygenated hemoglobin (HbO) chromophore or a deoxygenated hemoglobin (HbR) chromophore.

At 112, the one or more processors 102 further apply display processing to display the parametric maps, such as described in one or more of the patents/publications references herein.

Additionally or alternatively, the computing subsystem 100 may be configured to compute a collagen chromophore content in an ROI (e.g., the kidney). For example, the one or more processors 102 of the computing subsystem 100 generate an acoustic pressure data set 118 based on the OA return signal data 114. The acoustic pressure data 118 is associated with a corresponding sub-region of the ROI, wherein the acoustic pressure data 118 is dependent on a composition of collagen chromophores and non-collagen chromophores in the corresponding sub-region. At module 108, the computing subsystem 100 performs an unmixing operation by which the acoustic pressure data value for multiple individual specific locations in the ROI is decomposed into a collection of collagen and non-collagen chromophores. At 108, the one or more processors 102 further identify an extent of the non-collagen chromophore within each of one or more sub-regions and assign a value to a non-collagen chromophore factor based on the extent of the non-collagen chromophore within the corresponding one or more sub-regions. At 110, the one or more processors 102 compute a parametric map based on the amount of the collagen chromophore. The acoustic pressure data may include a collection of pressure data values representative of an acoustic response at corresponding positions throughout the sub-region of the ROI. At 112, the one or more processors 102 further apply display processing to display the parametric maps, such as described in one or more of the patents/publications references herein.

Principles Underlying Unmixing and Identification of Non-Hb Chromophore Extent

The optical absorption coefficient $\mu_a(\lambda, \mathbf{x})$, of a substance determines the amount of light that it can convert to heat. It is a function of the optical wavelength $\lambda$, and position vector x, as the molecular composition varies spatially within a volume of material. When a material is heated rapidly, pressure is generated. The amount of pressure $p_0$ is proportional to a material property called the Grüneisen parameter $\Gamma(\mathbf{x})$, which quantifies how added heat is converted to pressure. The radiant fluence $\Phi(\lambda, \mathbf{x})$ is the amount of light, at wavelength $\lambda$, that reaches position x during the entire time interval of the heating. The incident light is usually a short pulse of duration less than, say 200 ns, such that it satisfies a mechanical stress and a thermal spread condition. The acoustic pressure generated, immediately following the rapid heating, is then given by the equation:

$$p_0(\lambda, \mathbf{x}) = \mu_a(\lambda, \mathbf{x}) \ \Gamma(\mathbf{x}) \ \Phi(\lambda, \mathbf{x}) \qquad (1)$$

The generated pressure $p_0(\lambda, \mathbf{x})$, also referred to as the initial excess pressure, corresponds to the amount of OA return signal that is detected. Blood has a much stronger optical absorption coefficient $\mu_a$ as compared to the OAC of tissue. Accordingly, the acoustic pressure generated from blood, and thus the OA return signal produced by blood, is very strong relative to the acoustic pressure and OA return signal data produced by tissue. The large relative blood-based OA return signal results in high image contrast with respect to surrounding areas (sub-regions) of non-blood-based OA return signals. Accordingly, the computing subsystem generates the acoustic pressure data, for blood-based sub-regions, having larger amplitudes as compared to acoustic pressure data, for non-blood-based sub-regions.

The total hemoglobin mass concentration $c_{tHb}$, and oxygenation saturation levels $SO_2$, are clinically relevant parameters of interest for tissue lesion characterization. The total hemoglobin mass concentration $c_{tHb}$, and oxygenation saturation levels $SO_2$ can be determined from the Hb mass concentrations $c_{HbO}$ and $c_{HbR}$, by the equations:

$$c_{tHb} = c_{HbO} + c_{HbR} \qquad (7)$$

$$sO_2 = \frac{c_{HbO}}{c_{HbO} + c_{HbR}} \qquad (3)$$

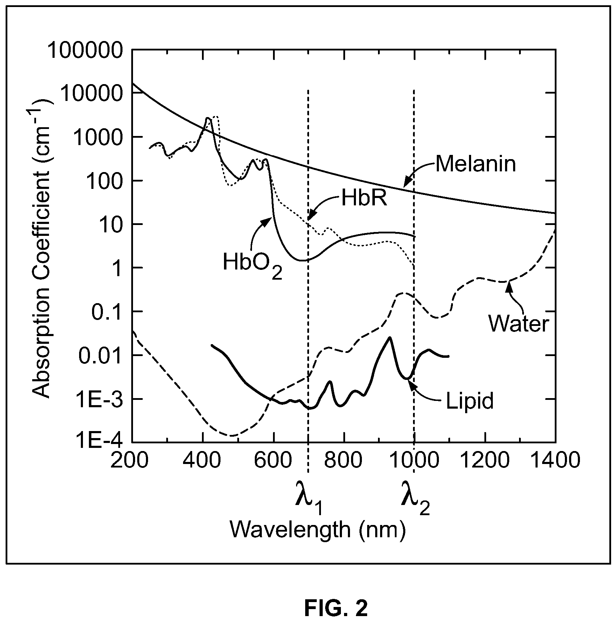

Figure 2 plots the optical absorption coefficient vs wavelength for the main chromophores in a patient's ROI that have a diagnostically relevant impact on results of OA imaging. As shown in figure 2, each of the five common chromophores in regions that imaged using OA imaging have separate and distinct spectra for optical absorption coefficients. The melanin chromophore exhibits an optical absorption coefficient that decreases in an approximately exponentially decaying manner over a range between $10000 cm^{-1}$ and $100 cm^{-1}$ along a wavelength spectrum of 200nm to 1400nm for the excitation laser light pulse(s). The water chromophore exhibits an optical absorption coefficient at around $0.1 cm^{-1}$ for an excitation wavelength of 200nm, dropping to $1E-4 cm^{-1}$ at an excitation wavelength of 500nm then increasing in an uneven undulating manner to $10 cm^{-1}$ at an excitation wavelength of 1400nm. The lipid chromophore exhibits an optical absorption coefficient at around $0.1 cm^{-1}$ for an excitation wavelength of 400nm, dropping to $1E-3 cm^{-1}$ at excitation wavelengths between 700nm and 800nm, then increasing in an uneven undulating manner to $0.11 cm^{-1}$ at an excitation wavelength of 1 100nm.

The Hb chromophores exhibit optical absorption coefficients that somewhat track/follow one another at excitation wavelengths between 200nm and 500nm. However, above 500nm, the optical absorption coefficients of HbO and HbR chromophores diverge from one another and exhibit different and somewhat opposed behaviors above 500nm, with a crossing point at around 800nm. In accordance with embodiments herein, the OA imaging system utilizes two excitation laser wavelengths with inverted ratios of the optical absorption coefficients as marked by the vertical dashed lines in figure 2, i.e., $\varepsilon_{hBO}(\lambda_1) < \varepsilon_{hBR}(\lambda_1)$ and $\varepsilon_{hBO}(\lambda_2) > \varepsilon_{hBR}(\lambda_2)$. The pair of excitation wavelengths are close to each other, so that their difference in optical attenuation (penetration depth) will not be too substantial and the optical fluence maps at the two wavelengths can be normalized by appropriate scaling factors. For example, at 780nm, the HbR and HbO chromophores exhibits optical absorption coefficients of $10 cm^{-1}$ and $1 cm^{-1}$. At 1060nm, the HbR and HbO chromophores exhibits optical absorption coefficients that have reversed to $1 cm^{-1}$ and $10 cm^{-1}$, respectively. By way of example, the relation between the OACs of HbO and HbR chromophores are substantially opposite or mirror values of one another at 780nm and 1060nm.

The optical absorption coefficient of a volume of tissue is a major contributor to the exponential decay rate of the incident light intensity as the light propagates through the tissue. For tissue that comprises of a mixture of chromophores, the overall optical absorption coefficient $\mu_{a,tissue}(\lambda)$ (in units of $cm^{-1}$) can be modeled as a weighted combination of each chromophore's absorption $\mu_{a,i}$ times its volume fraction $f_{v,i}$:

$$\mu_{a,tissue}(\lambda) = \sum_i f_{v,i} \, \mu_{a,i}(\lambda) \qquad (4)$$

For the biological tissue example cited above, the OAC for HbO, HbR, melanin, water and lipids can be stated as follows:

$$\mu_{a,tissue}(\lambda) = f_{v,HbO} \cdot \mu_{a,HbO}(\lambda) + f_{v,HbR} \cdot \mu_{a,HbR}(\lambda) + f_{v,melanim} \, \mu_{a,melanim}(\lambda) + f_{v,water} \, \mu_{a,water}(\lambda) + f_{v,lipids} \, \mu_{a,lipids}(\lambda) \qquad (5)$$

Optionally, the exponential optical attenuation due to absorption in a medium may be measured in base 10 (rather than the natural exponential "e"), in which case the optical absorption rate for a chromophore is referred to as its optical extinction coefficient $\varepsilon$ (in units of $cm^{-1} mol^{-1} L$). The optical absorption coefficient (OAC) and the optical extinction coefficient (OEC) are related by the following equation, where $C_i$ is the molar concentration (in units of mol $L^{-1}$, or M for Molarity) of the chromophore i:

$$\mu_{a,i}(\lambda) = \ln(10)\, C_i\, \varepsilon_i(\lambda) \qquad (6)$$

Alternatively, the molar concentration can be expressed as $C_i = c_i/MW_i$, in which ci is the mass concentration, and MWi is the molecular weight of the chromophore. In the example given, for normal whole blood in males, the average $C_{Hb}$ = 150g/L, and $MW_{Hb}$=64,458 g/mol, so that $C_{Hb}$= 2.33×10$^{-5}$ M. If a tissue $f_{v.blood}$ = 0.01 is assumed, then at $\lambda$=806 nm where $\varepsilon_{HbO}$= $\varepsilon_{HbR} \cong$ 818cm$^{-1}$ M$^{-1}$, $\mu_{a,Hb}(\lambda)$ = 0.04338 cm$^{-1}$ for both oxy- and deoxygenated Hb. The overall optical absorption coefficient in tissue can thus be written as follows:

$$\mu_{a,\,tissue}(\lambda) = \sum_i f_{v,i}\, \mu_{a,i}(\lambda) = \ln(10) \sum_i f_{v,i}\, c_i\, \varepsilon_i(\lambda)\, /\, MW_i \qquad (7)$$

Note that the contribution of chromophore i in the illuminated tissue is $\ln(10)\, f_{v,i}\, c_i\, \varepsilon_i(\lambda)/MW_i$. When equation (7) is combined with equation (1), for wavelength $\lambda$ at position x, this becomes:

$$p_o(\lambda,\, \mathbf{x}) = \ln(10)\, \Gamma(x)\, \Phi(\lambda,\, \mathbf{x}) \sum_i f_{v,i}\, c_i\, \varepsilon_i(\lambda)\, /\, MW_i \qquad (8)$$

The extent or contribution of the non-Hb chromophores may be combined into a single parameter, namely the OAC of "other" or non-Hb chromophores, $\mu^*_{a,other}(\lambda)=\ln(10)\Sigma_i\, c_i\, \varepsilon_i(\lambda)$. Substituting this into equation (8) yields the following equation.

$$\mu^*_{a,tissue}(\lambda) = \ln(10)\, [\, \acute{c}_{HbO}\, \varepsilon_{HBO}(\lambda) + \acute{c}_{HbR}\, \varepsilon_{HBR}(\lambda)\,] + \mu^*_{a,other}(\lambda) \qquad (9)$$

The OAC of "other" or non-HB chromophores $\mu^*_{a,other}(\lambda)$ can be interpreted as a bias. When the OAC of "other" or non-HB chromophores $\mu^*_{a,other}(\lambda)$ is added to the OAC of Hb chromophores, $\mu^*_{a,blood}(\lambda)$, the sum provides the total OAC of the composite tissue (e.g., all diagnostically relevant chromophores) in the sub-region, for which an acoustic pressure has been generated. Similarly, when the OAC for the composite tissue is know and the OAC for the non-Hb chromophores can be obtained, the OAC for the Hb chromophores can be calculated based on the following equation:

$$\mu^*_{a,blood}(\lambda) = \mu^*_{a,tissue}(\lambda) - \mu^*_{a,other}(\lambda) \qquad (10)$$

Optionally, among the non-Hb chromophores in biological tissue, water may be treated as the dominant chromophore for certain clinical OA applications. The OA pressure signal from HbO, HbR, water, plus one other generic chromophore i, can be written as follows, where $\acute{c}_i$ and $\varepsilon_i(\lambda)$ represent a fourth chromophore such as lipid and/or melanin:

$$p_o(\lambda,\, \mathbf{x}) = \ln(10)\, \Gamma(x)\, \Phi(\lambda,\, \mathbf{x})\, \{\, \acute{c}_{HbO}\, \varepsilon_{HBO}(\lambda) + \acute{c}_{HbR}\, \varepsilon_{HBR}(\lambda) + \acute{c}_{water}\, \varepsilon_{water}(\lambda) + \acute{c}_i\, \varepsilon_i(\lambda)\, \}$$

$$(12)$$

For blood, which is not a pure substance, but a suspension of blood cells in plasma, the standard values of the optical extinction coefficient $\varepsilon_i(\lambda)$ is based on normal hematocrit $H_o$=45% whole blood in major vessels (e.g., in a given volume of blood, 45% is red blood cells). But in clinical OA imaging, the key interests lie in Hb and/or $SO_2$ changes in the microvasculature that supply the lesion. In normal tissue, as the blood moves into the microvasculature (mv), the hematocrit percentage $Ht_{mv}$ will drop from the normal/standard percentage to a lower percentage (e.g., dropping from 45% into the 20% range). The degree of red blood cell drop is based on the patient's individual microvasculature. As long as the hematocrit percentage remains above a threshold (e.g., $Ht_{mv}$>20%), it can be assumed that the Hb chromophores (e.g., HbO, HbR, or a combination thereof) remains the diagnostically relevant dominant chromophores (optical absorber) within the volume of blood, as compared to other chromophores in blood (e.g., plasma/water). Based on the foregoing assumption, an approximation may be applied, such as by scaling down the optical extinction coefficient OEC by a factor corresponding to the ratio of the normal hematocrit percentage to the patient's actual/estimated hematocrit percentage in the ROI or sub-region (e.g., $Ht_{mv}/H_o$). Further, this OEC scaling factor can be incorporated into an apparent or overall molar concentration of HbO and HbR respectively, as follows:

$$\acute{c}_{HbO} = f_{v,blood}\,(Ht_{mv}/H_o)/\,MW_i\,c_{HbO} \qquad (9)$$

$$\acute{c}_{HbR} = f_{v,blood}\,(Ht_{mv}/H_o)/\,MW_i\,c_{HbR} \qquad (10)$$

Even though the Hb chromophores may vary in concentration across sub-regions of the ROI, in accordance with some embodiments, the extent of Hb chromophore contributions, for all or a collection of sub-regions of the ROI, may be consolidated into the overall variables $c_{HbO}$ and $c_{HbR}$. The Hb chromophore contributors may be consolidated in embodiments where it is not practical or desired to parse out changes in $f_{v,blood}$ (e.g., vascularization changes relative to a tissue volume), $Ht_{mv}$ (hematocrit changes in microvasculature) or $c_{HbO}$ and $c_{HbR}$ (Hb molar concentration changes within the red blood cells). However, for a microvasculature with a hematocrit percentage $Ht_{mv}$ that reduces below 20%, the simple scale factor $Ht_{mv}/Ho$ approximation may become sufficiently inaccurate to affect diagnostic relevance of the scale factor (e.g., impact the diagnostic analysis). Additionally or alternatively, once a microvasculature of a sub-region exhibits a hematocrit percentage below 20%, the sub-region may no longer qualify as "blood" or a "blood vessel." Hence, in accordance with new and unique aspects herein, embodiments may identify the sub-region that exhibits an unduly low hematocrit percentage and re-classify the sub-region as tissue that has a large extent of the water/plasma non-Hb chromophore. Additionally or alternatively, embodiments may reduce the blood volume fraction for the sub-region $f_{v,blood}$. The use of the above overall variables 6HbO and cHbR could accommodate either interpretation. Ultimately, at least one goal in clinical OA imaging is to highlight changes in the overall Hb levels relative to the tissue baseline that may be indicative of angiogenesis and/or hypoxia.

In comparison to blood Hb, other common chromophores in biological tissue such as water, lipid and melanin occur in relatively pure form, such that their overall molar concentrations in tissue can be simply expressed as:

$$\acute{c}_i = f_{v,i}\,c_i\,/\,MW_i \qquad (11)$$

The above derivation can be generalized by defining $\acute{c}_{HbO}$ and $\acute{c}_{HbR}$ as overall HbO and HbR concentrations respectively. The solution can be obtained by simply inverting the extinction coefficient matrix:

$$\begin{bmatrix} \acute{c}_{HbO} \\ \acute{c}_{HbR} \end{bmatrix} = 1/\ln(10) \begin{bmatrix} \varepsilon_{HbO}(\lambda_1) & \varepsilon_{HbR}(\lambda_1) \\ \varepsilon_{HbO}(\lambda_2) & \varepsilon_{HbR}(\lambda_2) \end{bmatrix}^{-1} \begin{bmatrix} \mu^*_{a,blood}(\lambda_1) \\ \mu^*_{a,blood}(\lambda_2) \end{bmatrix} \qquad (12)$$

The solution of the two-wavelength OA system matrix that accounts for non-Hb chromophore contributions can then be written as follows:

$$\begin{bmatrix} \acute{c}_{HbO} \\ \acute{c}_{HbR} \end{bmatrix} = 1/\ln(10) \begin{bmatrix} \varepsilon_{HbO}(\lambda_1) & \varepsilon_{HbR}(\lambda_1) \\ \varepsilon_{HbO}(\lambda_2) & \varepsilon_{HbR}(\lambda_2) \end{bmatrix}^{-1} \begin{bmatrix} \mu^*_{a,blood}(\lambda_1) \\ \mu^*_{a,blood}(\lambda_2) \end{bmatrix}$$

$$\text{where} \quad \begin{bmatrix} \mu^*_{a,blood}(\lambda_1) \\ \mu^*_{a,blood}(\lambda_2) \end{bmatrix} = \begin{bmatrix} \mu^*_{a,tissue}(\lambda_1) \\ \mu^*_{a,tissue}(\lambda_2) \end{bmatrix} - \begin{bmatrix} \mu^*_{a,other}(\lambda_1) \\ \mu^*_{a,other}(\lambda_2) \end{bmatrix}$$

$$\text{in which} \quad \begin{bmatrix} \mu^*_{a,tissue}(\lambda_1) \\ \mu^*_{a,tissue}(\lambda_2) \end{bmatrix} = \begin{bmatrix} p_0(\lambda_1) / (\Gamma(x)\,\Phi^*(\lambda_1)) \\ p_0(\lambda_2) / (\Gamma(x)\,\Phi^*(\lambda_2)) \end{bmatrix}$$

$$(13)$$

## Embodiments

Next, embodiments are described for a two-wavelength OA system utilizing the foregoing chromophore concepts.

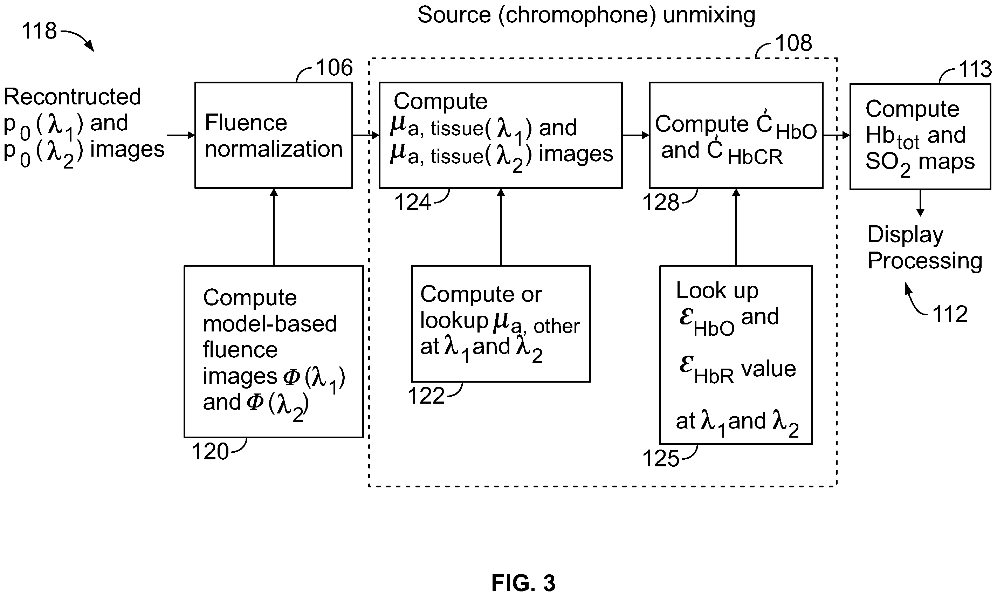

Figure 3 illustrates a collection of modules implemented by the computing subsystem 100 in more detail, and in particular, the chromophore unmixing sub-system implemented in accordance with embodiments herein. At module 106, the one or more processors of the computing subsystem 100 perform fluence normalization based on computer model-based fluence images $\Phi(\lambda 1)$ and $\Phi(\lambda 2)$ (which are obtained at 120) associated with the first and second laser light pulses having first and second predominant wavelengths (e.g., 780nm and 1060nm).

At module 120, the one or more processors may obtain predefined model-based fluence images $\Phi(\lambda 1)$ and $\Phi(\lambda 2)$ from memory. Additionally or alternatively, at module 120, the one or more processors may calculate the model-based fluence images $\Phi(\lambda 1)$ and $\Phi(\lambda 2)$ based on predefined characteristics of base images for a patient population. Additionally or alternatively, at module 120, the one or more processors may calculate the model-based fluence images $\Phi(\lambda 1)$ and $\Phi(\lambda 2)$ based on the specific current patient for which the OA images are obtained. In one embodiment, patient data may be used to adaptively adjust the fluence model parameters such as optical attenuation coefficient, for model-based fluence correction. As another example, one or more processors may calculate the model-based fluence images based on characteristics of interest from the ROI of the current patient. For example, the one or more processors may obtain tissue sub-types associated with sub-regions of the ROI of the patient and based thereon calculate the model-based fluence images $\Phi(\lambda 1)$ and $\Phi(\lambda 2)$. The tissue sub-types may be input by a clinician such as while reviewing diagnostic images of the ROI. The tissue sub-type(s) may be determined based on patient data entered in the patient's medical record (e.g., age, weight, specific descriptions of the patient's anatomy for the ROI). The diagnostic images may be obtained through various imaging modalities, such as ultrasound, CT, X-ray, SPECT, PET, MRI and the like. For example, the images, to be analyzed (via computer or via clinician) may be obtained utilizing the methods and systems in one or more of the following, all of which are expressly incorporated herein by reference in their entireties:

US20220370037A1, titled "VASCULAR TISSUE CHARACTERIZATION DEVICES, SYSTEMS, AND METHODS" published 2022-11-24; EP2208465A1, titled "ULTRASONOGRAPHIC DEVICE, OPERATION METHOD THEREOF, AND ULTRASONOGRAM DIAGNOSIS PROGRAM" published 2010-07-21; US20220240890A1, titled "SYSTEM AND METHOD FOR MULTIPATH PROCESSING OF IMAGE SIGNALS" published 2022-08-04; US20140288425A1, titled "APPARATUS AND METHOD FOR PROVIDING ELASTICITY INFORMATION" published 2014-09-25; US20170090571A1, titled "SYSTEM AND METHOD FOR DISPLAYING AND INTERACTING WITH ULTRASOUND IMAGES VIA A TOUCHSCREEN" published 2017-03-30; WO2018046095A1, titled "ENDOSCOPE AND METHOD FOR OPERATING AN ENDOSCOPE" published 2018-03-15; US20220225967A1, titled "ULTRASOUND IMAGING SYSTEM WITH A NEURAL NETWORK FOR DERIVING IMAGING DATA AND TISSUE INFORMATION" published 2022-07-21; EP3740132A1, titled "AUTOMATED PATH CORRECTION DURING MULTI-MODAL FUSION TARGETED BIOPSY" published 2020-11-25; US20210113190A1, titled "ULTRASOUND LESION ASSESSMENT AND ASSOCIATED DEVICES, SYSTEMS, AND METHODS" published 2021-04-22; WO2021069216A1, titled "VASCULAR TISSUE CHARACTERIZATION DEVICES, SYSTEMS, AND METHODS" published 2021-04-15; WO2022069377A1, titled "RENDERING OF B-MODE IMAGES BASED ON TISSUE DIFFERENTIATION" published 2022-04-07; US11452503B2, titled "SHEAR WAVE IMAGING BASED ON ULTRASOUND WITH INCREASED PULSE REPETITION FREQUENCY", published 2022-09-27; US20220225967A1 , titled "ULTRASOUND IMAGING SYSTEM WITH A NEURAL NETWORK FOR DERIVING IMAGING DATA AND TISSUE INFORMATION", published 2022-07-21; US10338203B2, titled "CLASSIFICATION PREPROCESSING IN MEDICAL ULTRASOUND SHEAR WAVE IMAGING", published 2019-07-02; US9465090B2, titled "METHOD OF MAGNETIC RESONANCE-BASED TEMPERATURE MAPPING", published 2016-10-11; FR2934054B1, titled "SHEAR WAVE IMAGING", published 2015-01-16; and EP3787519B1, titled "SHEAR WAVE AMPLITUDE RECONSTRUCTION FOR TISSUE ELASTICITY MONITORING AND DISPLAY", published 2021-10-13.

The diagnostic images may be obtained prior to or at the time of a current OA imaging examination and analyzed by the clinician prior to or at the time of the OA imaging examination. The clinician may enter the tissue sub-types into the patient's medical record. Additionally or alternatively, the clinician may enter the tissue sub-types into meta-data associated with the diagnostic image(s), where the tissue sub-types are stored with the diagnostic imaging data in a patient file in accordance with the Digital Imaging and Communications in Medicine (DICOM) format.

Additionally or alternatively, the tissue sub-types may be determined based on computer-based segmentation and analysis of diagnostic images of the ROI. The computer-based analysis of the diagnostic images may be performed by the computing subsystem 100 or by another remote computing system (e.g., a separate diagnostic imaging system, a Picture archiving and communication systems (PACS) imaging system, a medical network server and the like). The computer-based analysis may be applied to one or more ultrasound images obtained by the OA imaging system, and/or diagnostic images from another imaging system (e.g., another UL system, and/or CT, X-ray, PET, SPECT, MRI imaging systems, etc.). When based on ultrasound images, the tissue sub-types may be determined from various types of ultrasound data, such as B-mode (gray-scale) images, Doppler images, color flow images, elastography images, shear-wave imaging, and the like.

The model-based fluence images are passed to the fluence normalization calculation at 106, where the one or more processors apply fluence normalization to the acoustic pressure data and/or reconstructed OA images. For example, the fluence normalization adjusts the OA pressure estimate $p_o(\lambda 1, x)$ for each pixel to compensate for wavelength-dependent optical attenuation and scattering, as a function of depth, experiences by the received OA signals. The attenuation and scattering effect an amplitude of the received OA signals in connection with each point (voxel) in the region of interest. In the embodiment of Figure 3, the fluence normalization may be based on standard optical attenuation and scattering fluence image models or based on models that are developed based on specific tissue sub-types of the current individual patient being examined. The fluence-adjusted acoustic pressure data is passed to the chromophore unmixing module 108.

At module 108, the one or more processors implements modules 122-128. The discussion hereafter for the operations at 122-128 is provided in the context of a sub-region, however, it is recognized that the operations may be implemented for at least one of: i) multiple sub-regions throughout the ROI, ii) multiple positions throughout one sub-region, iii) multiple positions throughout multiple sub-regions, and/or once for a complete ROI (e.g., if treating the ROI as one sub-region). The operations at 122-128 are implemented in connection with at least first and second sets of OA return signal data that are returned in response to first and second laser light pulses (e.g., one OA return signal data set based on a laser excitation having a dominant wavelength at 780nm, and one OA return signal data set based on a laser excitation having a dominant wavelength at 1060nm). Additionally or alternatively, the operations at 122-128 may be implemented in connection with third, fourth or more sets of OA return signal data that are returned in response to third, fourth or more laser light pulses having third, fourth or more dominant wavelengths.

Optionally, the operations at 122-128 may be implemented for different sub-regions based on different first and second excitation laser light pulses having dominant first and second wavelengths. For example, first and second excitation laser light pulses having dominant first and second wavelengths may be utilized to excite sub-regions near the skin surface, while the first and/or second excitation laser light pulses and/or third or even fourth excitation laser light pulses having different dominant wavelengths may be used for sub-regions deeper below the surface. Additionally or alternatively, laser light pulses with different dominant wavelengths may be used for sub-regions having different compositions.

At module 122, the one or more processors identify the extent of the non-Hb chromophore within the sub-region. At module 122, once the extent of the non-Hb chromophore within the sub-region is identified, the one or more processors assign a value to a non-Hb chromophore factor based on the extent of the non-Hb chromophore within the sub-region. As non-limiting examples, the non-Hb chromophore factor(s) may correspond to at least one of: i) an optical absorption coefficient of the non-Hb chromophore in the sub-region, ii) an optical extinction coefficient of the non-Hb chromophore in the sub-region, iii) a molar concentration of the non-Hb chromophore in the sub-region, iv) a mass concentration of the non-Hb chromophore in the sub-region, or iv) a fractional volume of the non-Hb chromophore in the sub-region. For example, the extent of a non-Hb chromophore and the value assigned to the corresponding non-Hb chromophore factor may be obtained from a look-up table. For example, a look-up table may be utilized as follows to identify the extent and assign the value:

**TABLE 1**

| Tissue Type and/or Subtype | Extent of non-Hb Chromophore | Value for non-Hb Chromophore Factor | Optical Absorption Coefficient | Optical Extinction Coefficient | Molar Concentration, Mass Concentration |
|---|---|---|---|---|---|
| Fatty Tissue (combination of water, lipid, melanin) | High/Med/Low Volume Fraction | $f_{v,\text{Fatty -non-Hb}}$ | $\mu_{a,\text{Fatty - non-Hb}}(\lambda 1)$ <br> $\mu_{a,\text{Fatty - non-Hb}}(\lambda 2)$ | $\varepsilon_{a,\text{Fatty - non-Hb}}(\lambda 1)$ <br> $\varepsilon_{a,\text{Fatty - non-Hb}}(\lambda 2)$ | $MW_{,\text{Fatty -non-Hb}}$ <br> $C_{,\text{Fatty -non-Hb}}$ |
| Non-Fatty Tissue (combination of water, lipid, melanin) | High/Med/Low Volume Fraction | $f_{v,\text{non-Fatty- non-Hb}}$ | $\mu_{a,\text{Non-Fatty - non-Hb}}(\lambda 1)$ <br> $\mu_{a,\text{Non-Fatty - non-Hb}}(\lambda 2)$ | $\varepsilon_{a,\text{Non-Fatty-non-Hb}}(\lambda 1)$ <br> $\varepsilon_{a,\text{Non-Fatty-non-Hb}}(\lambda 2)$ | $MW_{,\text{non-Fatty- non-Hb}}$ <br> $C_{,\text{non-Fatty- non-Hb}}$ |
| Water | High/Med/Low Volume Fraction | $f_{v,\text{water}}$ | $\mu_{a,\text{water}}(\lambda 1)$ <br> $\mu_{a,\text{water}}(\lambda 2)$ | $\varepsilon_{a,\text{water}}(\lambda 1)$ <br> $\varepsilon_{a,\text{water}}(\lambda 2)$ | $MW_{\text{water}}$ <br> $C_{\text{water}}$ |
| Lipid | High/Med/Low Volume Fraction | $f_{v,\text{lipid}}$ | $\mu_{a,\text{lipid}}(\lambda 1)$ <br> $\mu_{a,\text{lipid}}(\lambda 2)$ | $\varepsilon_{a,\text{lipid}}(\lambda 1)$ <br> $\varepsilon_{a,\text{lipid}}(\lambda 2)$ | $MW_{\text{lipid}}$ <br> $C_{\text{lipid}}$ |
| Melanin | High/Med/Low Volume Fraction | $f_{v,\text{melanin}}$ | $\mu_{a,\text{melanin}}(\lambda 1)$ <br> $\mu_{a,\text{melanin}}(\lambda 2)$ | $\varepsilon_{a,\text{melanin}}(\lambda 1)$ <br> $\varepsilon_{a,\text{melanin}}(\lambda 2)$ | $MW_{\text{melanin}}$ <br> $C_{\text{melanin}}$ |

To identify the extent, the one or more processors may, at least one of: i) determine a volume fraction of the non-Hb chromophore in the sub-region, ii) classify the sub-region into one or more tissue types/sub-types (e.g., non-fatty tissue, fatty tissue, blood, water, lipid dominated, melanin dominated), or iii) determine a concentration of the non-Hb chromophore in the sub-region. The extent of the non-Hb chromophore may be expressed in various manners. For example, the extent of a non-Hb chromophore may be set as high, medium, or low, set on a scale of 1-10, set on a normalized scale, etc. A single composite "extent" may be identified for all non-Hb chromophores collectively, or separate chromophore-specific extents may be assigned for water, lipids, melanin or any combination thereof.

The extent of a non-Hb chromophore may be based on tissue type/subtype. For example, a sub-region may be classified as non-fatty tissue which is in turn assigned a low value for the distribution or concentration of water, while a sub-region classified as fatty tissue is assigned a high value for water. As another example, sub-regions classified as lipid dominated or melanin dominated may be assigned high values for the lipid and/or melanin chromophore with a lower value for the water chromophore. The basis for classification of a sub-region may be the same or different from the computer-based analysis or clinician feedback discussed above in connection with module 120 for computing model-based fluence. For example, the tissue sub-types may be input by a clinician such as while reviewing diagnostic images of the ROI. Additionally or alternatively, the tissue types/subtypes may be determined based on computer-based segmentation and analysis of diagnostic images of the ROI. Once the tissue type/subtype is known, a lookup table (e.g., Table 1) may be accessed to obtain a value for one or more non-Hb chromophore factors. For example, the lookup table may contain different values, based on the tissue type/subtype, for a volume fraction of the non-Hb chromophore in the sub-region. For example, fatty tissue will have a higher volume fraction of water than non-fatty tissue. A sub-region near the surface may have a higher volume fraction of melanin than a sub-region deeper within the ROI. Additionally or alternatively, the lookup table may contain different values, based on the tissue type/subtype, for molar concentration of the non-Hb chromophore in the sub-region, mass concentration of the non-Hb chromophore in the sub-region, and the like. The lookup table may also contain, as non-Hb chromophore factor, at least one of: i) an optical absorption coefficient of the non-Hb chromophore in the sub-region and/or ii) an optical extinction coefficient of the non-Hb chromophore in the sub-region. While water may have one OAC and one OEC regardless of tissue type/subtype, the classification may classify a tissue type/subtype to be separately water or classify a tissue type/subtype to be "fatty tissue" which has a combination of water and other chromophores. Hence, the OAC and OEC of fatty tissue will differ from the OAC and OEC of isolated water.

Additionally or alternatively, tissue need not be classified to determine the extent of non-Hb chromophores. For example, a distribution or concentration of non-Hb chromophores may be entered by a clinician for one or more sub-regions. The clinician entered distribution or concentration may be numeric value, a percentage or a relative rank (e.g., high, medium, low). As another example, the distribution or concentration of the non-Hb chromophore may be automatically calculated by one or more processors. For example, diagnostic images of the ROI may be segmented and analyzed automatically by the computing system or another computing system to determine the distribution or concentration of one or more non-Hb chromophores.

The one or more processo rs assign a value to a non-Hb chromophore factor based on the extent of the non-Hb chromophore within the sub-region. A single value may be assigned for a "collective" non-Hb chromophore factor, or separate values may be assigned for corresponding individual water, lipid and/or melanin chromophore factors. For example, the one or more processors may determine the value for the non-Hb chromophore factor based on at least one of: i) an optical absorption coefficient of the non-Hb chromophore in the sub-region, ii) an optical extinction coefficient and molar concentration of the non-Hb chromophore in the sub-region, iii) a mass concentration and molecular weight of the non-Hb chromophore in the sub-region, or iv) a fractional volume of the non-Hb chromophore in the sub-region. As an example, the value for a distribution or concentration may be determined based on the molar concentration, mass concentration and/or molecular weight of the chromophore of interest (e.g., using equations 6 and/or 7 but for the chromophore(s) of interest).

The value(s) for the non-Hb chromophore factor are provided from module 122 to module 124. As noted above, the one or more processors are further configured to repeat the generate, identify, assign and compute operations for at least one of: i) multiple sub-regions throughout the ROI, ii) multiple positions throughout the subregion, or iii) multiple positions throughout multiple sub-regions throughout the ROI. When the ROI includes first and second sub-regions that have different first and second compositions, the one or more processors identify first and second extents of the non-Hb chromophore within the first and second sub-regions, respectively, and, based thereon, assign first and second values to the non-Hb chromophore factor for the first and second sub-regions, respectively. The one or more processors repeat the operations at module 122 in connection with at least first and second excitation laser light pulses having dominant first and second wavelengths.

At module 124, the one or more processors calculate the overall OAC for the sub-region, $\mu_{a,tissue}(\lambda 1, x)$ and $\mu_{a,tissue}(\lambda 2, x)$, in connection with each excitation laser light pulse. As noted herein, in connection with equation (10), the overall OAC for all chromophores in a sub-region is referred to as the tissue OAC $\mu_{a,tissue}(\lambda, x)$. The tissue OAC $\mu_{a,tissue}(\lambda, x)$ represents the sum of OAC for the Hb chromophores $\mu^*_{a,blood}(\lambda, x)$ and the OAC for the non-Hb chromophores

$\mu^*_{a,other}(\lambda,x)$, in connection with the laser having the current dominant wavelength $\lambda$. The tissue OAC is calculated based on the acoustic pressure data $p_o(\lambda,x)$, the $\Gamma(x)$ Grüneisen parameter, and $\Phi(\lambda, x)$ radiant fluence, namely $\mu_{a,tissue}(\lambda,x)$ = $p_o(\lambda,\mathbf{x})/[\Gamma(x) * \Phi(\lambda, x)]$. The variable x represents a current sub-region or a pixel/voxel in a sub-region, and can be included or omitted, depending on the level of specificity desired for the calculation.

Once the tissue OAC $\mu_{a,tissue}(\lambda,x)$ is known, the one or more processors can calculate the blood OAC $\mu^*_{a,blood}(\lambda)$ (at module 124) as the difference between the tissue OAC $\mu_{a,tissue}(\lambda,x)$ and the OAC of the non-Hb chromophores $^*_{a,other}(\lambda,x)$.

At module 126, the one or more processors obtain the OECs for oxygenated and de-oxygenated blood HbO and HbR for each excitation laser wavelength. For example, the one or more processors may access a lookup table to obtain the OEC for oxygenated blood $\varepsilon_{HbO}(\lambda 1)$ and $\varepsilon_{HbO}(\lambda 2)$ at first and second laser wavelengths, and the OEC for de-oxygenated blood $\varepsilon_{HbR}(\lambda 1)$ and $\varepsilon_{HbR}(\lambda 2)$ at first and second laser wavelengths (collectively the "blood OECs"). The blood OECs are supplied to module 128.

At module 128, the one or more processors use the blood OECs and blood OACs to compute the concentration of oxygenated and de-oxygenated blood cHbO and cHbR in the sub-region. The concentration may be expressed in terms of molar concentration. For example, the concentration of oxygenated and de-oxygenated blood cHbO and cHbR may be calculated based on the blood OAC and blood OEC for each excitation laser wavelength, utilizing equation (12).

The concentration of oxygenated and de-oxygenated blood cHbO and cHbR are passed from module 128 to module 113. At module 113, the one or more processors compute the total hemoglobin mass concentration map ($c_{tHb}$ = $c_{HbO}$ + $c_{HbR}$) and oxygenation saturation level map $SO_2$, such as described in the patents and publications referenced herein. Thereafter, the one or more processors perform display processing 112 upon the maps.

The computing subsystem of Figure 3 may be implemented in various computing environments, as a stand-alone computer, on one or more servers and the like. Additionally or alternatively, the computing system of Figure 3 may be implemented within an OA imaging system.

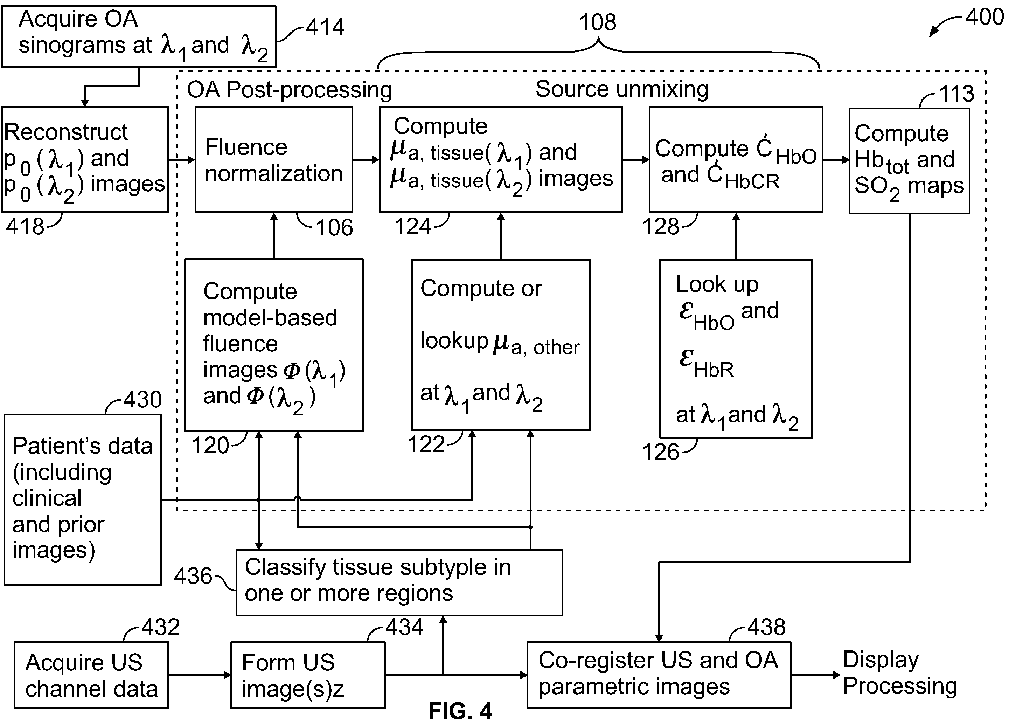

Figure 4 illustrates a block diagram of an OA imaging system 400 that implements the computing subsystem of Figure 3 in accordance with embodiments herein. The OA imaging system 400 includes memory and one or more processors configured to execute program instructions stored in the memory. The one or more processors generate an acoustic pressure data set 418 based on the OA return signal data 414, such as in the manner discussed in connection with Figure 1. As The acoustic pressure data set 418 is associated with a corresponding sub-region of the ROI, wherein the acoustic pressure data set 418 is dependent on a composition of Hb chromophores and non-Hb chromophores in the corresponding sub-region. In accordance with embodiments herein, the one or more processors may generate an OA image (as denoted at 418 as "reconstructed images") from the OA return signal data 414, in which case the acoustic pressure data set 418 for the ROI includes and/or represents the OA image. The source unmixing module 108 performs the operations, and interacts with modules 106 and 113, as discussed in connection with Figures. 1 and 3.

The one or more processors manage transmit and receive operations of an ultrasound probe to acquire ultrasound (US) data (at module 432). The acquired raw US data is processed to form US images (at module 434). The US images are supplied to modules 436 and 438. At module 436, the one or more processors segment the US image(s) and classify tissue types/subtypes within one or more sub-regions. The classification may be implemented as a manual process by a clinician and/or a computer automated manner. At module 438, the one or more processors co-register the US image(s) and OA parametric maps(s), as described in more detail in the patents and publications referenced herein.

The OA imaging system 400 stores, in memory, patient data 430, including clinical images and prior images. The images may be from various modalities, such as MRI, PET, SPECT, X-ray, CT, US, etc. In another embodiment, the one or more processors adaptively adjust the value(s) assigned to the non-Hb chromophore factor(s) $\mu_{a,other}(\lambda)$ based on the patient data. As an example, for breast tissue, the tissue subtype may be classified into several categories in terms of fibrous and fat content, and a look-up-table is used to store the corresponding values for the non-Hb chromophore factor(s) $\mu_{a,other}(\lambda)$. As another example, for the prostate gland, prior MRI images may be leveraged to classify different prostate zones with different volume fractions for the water chromophore.

The modules 434 and 436 utilize patient data, pathology data, and/or tissue classification information to provide or update parameters utilized by modules 120 and 122 (e.g., the model-based fluence image calculation, identification of chromophore extent and/or assignment of values to the non-Hb chromophore factor(s). In accordance with embodiments, the optical extinction coefficients of HbO and HbR are treated as constant (e.g., not varying with patient data). Additionally or alternatively, embodiments can extend the use of patient data to select the preferred optical extinction coefficient values.

In accordance with embodiments, the tissue subtype information may be extracted from anatomical B-mode US image data. As an example, a qualitative grading of the glandular tissue component in breast US images can serve as a biomarker for breast cancer. The glandular tissue component can be simply classified as minimal, mild, moderate or marked, based on visual judgment by the clinician or based on computer automated analysis. Optionally, different tissue subtypes may be mapped to different tissue parameters (e.g., fractional water volume, or optical attenuation coefficient) as soft priors for improved spectral unmixing. As another example, an unusually echogenic region in the B-mode image indicative of higher fat content may justify use of a higher volume fraction for the lipid chromophore and a lower volume fraction for the water chromophore. As a third example, an estimate of the volume of a prostate or thyroid gland from the US image may be used to set the volume fraction for the blood chromophores and/or the volume fraction for the water chromophore in the region being illuminated for OA functional imaging.

In another embodiment, the US data may include US color Doppler flow and/or elastography images which by themselves could also complement the functional data from OA imaging. The corresponding tissue subtype information would be based on blood flow and tissue stiffness characteristics, respectively, that are obtained based on the color Doppler flow and elastography images. For example, US elastography images may be used to classify the tissue as soft, normal, or stiff. A stiff rating may be associated with a lower volume fraction for water and/or a higher optical scattering coefficient with respect to average values otherwise assigned for a given tissue type or organ.

In another embodiment, embodiments may extract information from US image data by displaying the US images to a clinician. The clinician visually judges the US images and manually enters, into a look-up table, one or more index values for corresponding Hb and non-Hb chromophore factors. Additionally or alternatively, embodiments may implement an automated image analysis method, in which one or more processors perform classical image segmentation and/or modern machine learning methods (including deep learning). A machine learning approach can be advantageous because recognition of tissue subtypes would only require training data sets from normal human subjects, which are much easier to collect than labeled datasets representing disease cases.

In another embodiment, both patient data and US image data may be used in combination for the tissue classification operation. For example, a high BMI coupled with an unusually echogenic B-mode image may indicate a higher volume fraction for the lipid chromophore and a lower volume fraction for the water chromophore. In another embodiment, prior non-US medical image data (e.g., MRI) or prior US image data can be used in combination with the newly acquired US image data from the hybrid US-OA imaging system for the tissue classification step.

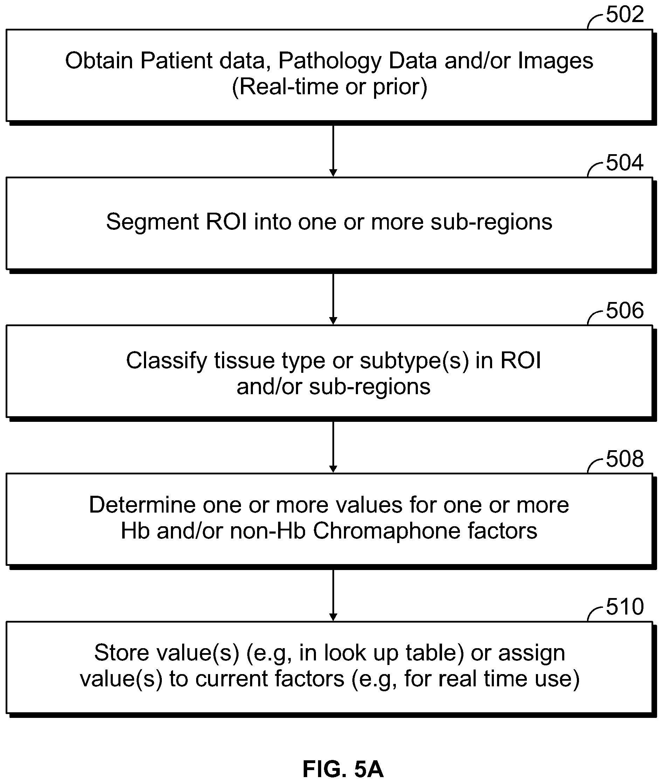

Figure 5A illustrates a method for determining chromophore factor values in accordance with embodiments herein. The method may be performed by one or more processors of the various subsystems, OA imaging systems and computing systems of Figures 1-8, as well as by other computing devices, servers and the like.

At 502 the one or more processors obtain patient data, pathology data and/or images associated with a patient. The patient data, pathology data and/or images may be previously acquired or acquired in real-time as part of (or contemporaneous with) the OA imaging examination. At 504, the one or more processors segment the ROI into one or more subregions. The segmentation may be based on manual designations entered by the clinician and/or automatically by the computer. At 506, the one or more processors obtain classifications for the tissue type(s) and/or tissue subtype(s). The classification(s) may be entered by a clinician and/or automatically determined by the processor(s). At 508, the one or more processors determine one or more values for one or more Hb and/or non-Hb chromophore factors. At 510, the one or more processors store the value(s) for the one or more Hb and/or non-Hb chromophore factors in memory, such as in a lookup table. Additionally or alternatively, the one or more processors assign the value(s) for the one or more Hb and/or non-Hb chromophore factors. The values may be assigned in real-time while performing an OA imaging examination. Additionally or alternatively, the one or more processors may save the value(s) for the one or more Hb and/or non-Hb chromophore factors in the patient's medical record.

Distinguishing Between Chromophores

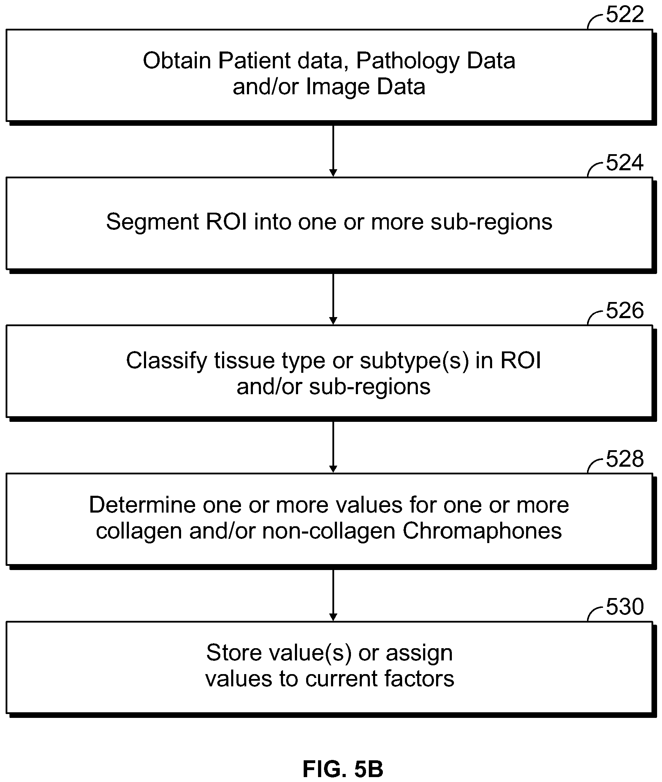

Figure 5B illustrates a method for determining chromophore factor values in accordance with alternative embodiments herein. The embodiment of Figure 5B distinguishes the collagen chromophore from other non-collagen chromophores. For example, it may be desirable to utilize OA imaging of kidney fibrosis for assessing pretransplant kidney quality. Before transplanting a kidney it is desirable to assess the quality of the kidney to be transplanted. Hysi et al. describe a general OA imaging technique to directly image collagen, Hysi, Eno, et al. "Photoacoustic imaging of kidney fibrosis for assessing pretransplant organ quality," JCI insight 5.10 (2020), incorporated herein by reference in its entirety. OA imaging can be utilized to noninvasively quantify whole kidney fibrosis burden, as well as capture intrarenal variations in collagen content. In accordance with new and unique aspects herein, the methods and systems herein can be applied to improve stage III post-processing to improve the specificity and sensitivity of the OA imaging system with respect to collagen within sub-regions of an ROI. By improving the specificity and sensitivity of the OA imaging system with respect to collagen in OA images, methods and systems herein enable better assessment of kidney fibrosis burden which in turn improves assessment of transplant candidate kidneys. Accordingly, embodiments herein improve treatment and prophylaxis (e.g., actions taken to prevent kidney disease) kidney disease.

The method of Figure 5B may be performed by one or more processors of the various subsystems, OA imaging systems and computing systems of Figures 1-8, as well as by other computing devices, servers and the like. The embodiment of Figure 5B seeks to compute identify an extent of the at least one non-collagen chromophore within the sub-region, assign a value to a non-collagen chromophore factor based on the extent of the at least one non-collagen chromophore within the sub-region; and compute an amount of at least one of collagen chromophore in the sub-region based on the acoustic pressure data and the value assigned to the non-collagen chromophore factor.

With reference to Figure 5B, at 522 the one or more processors obtain patient data, pathology data and/or images associated with a patient. The patient data and pathology data may be previously stored in connection with the patient's medical record and/or entered by a clinician at the time of the OA imaging processes. For example, the pathology data may include test results from prior biopsies or other samples taken from a patient's tissue of interest and/or a patient's fluids (e.g., blood, urine, etc.). Additionally or alternatively, the pathology data may include clinician reports generated based on the biopsies or other studies of the patient's tissue and/or fluids. Ultrasound images or images from other diagnostic imaging modalities (e.g., MRI, CT, PET, SPECT) may be previously acquired or acquired in real-time as part of (or contemporaneous with) the OA imaging examination.

At 524, the one or more processors segment the ROI into one or more subregions. The segmentation may be based on manual designations entered by the clinician and/or automatically by the computer. For example, a clinician may designate certain points or portions of a kidney to be treated as a separate sub-region. Additionally or alternatively, the one or more processors may automatically analyze the images (e.g., ultrasound images, MRI images, etc.) and segment the images into corresponding sub-regions. At 526, the one or more processors obtain classifications for the tissue type(s) and/or tissue subtype(s). The classification(s) may be entered by a clinician and/or automatically determined by the processor(s). At 528, the one or more processors determine one or more values for one or more collagen and/or non-collagen chromophore factors. At 530, the one or more processors store the value(s) for the one or more collagen and/or non-collagen chromophore factors in memory, such as in a lookup table. Additionally or alternatively, the one or more processors assign the value(s) for the one or more collagen and/or non-collagen chromophore factors. The values may be assigned in real-time while performing an OA imaging examination. Additionally or alternatively, the one or more processors may save the value(s) for the one or more collagen and/or non-collagen chromophore factors in the patient's medical record.

Once the amount of collagen chromophore is known, various OA images/maps may be generated to highlight the collagen content and/or collagen burden within one or more sub-regions and/or the ROI in the OA images. The amount of collagen chromophore is used to improve stage III post-processing to improve the specificity and sensitivity of the OA imaging system with respect to collagen within sub-regions of an ROI. By improving the specificity and sensitivity of the collagen in the OA images, methods and systems herein enable better assessment of kidney fibrosis burden which in turn improves assessment of transplant candidate kidneys. Accordingly, embodiments herein improve treatment and prophylaxis (e.g., actions taken to prevent kidney disease) kidney disease.

Optionally, in accordance with alternative embodiments, the methods and systems herein may process a lipid chromophore as the chromophore of interest, with all other chromophores as chromophores not of interest.

System Overview

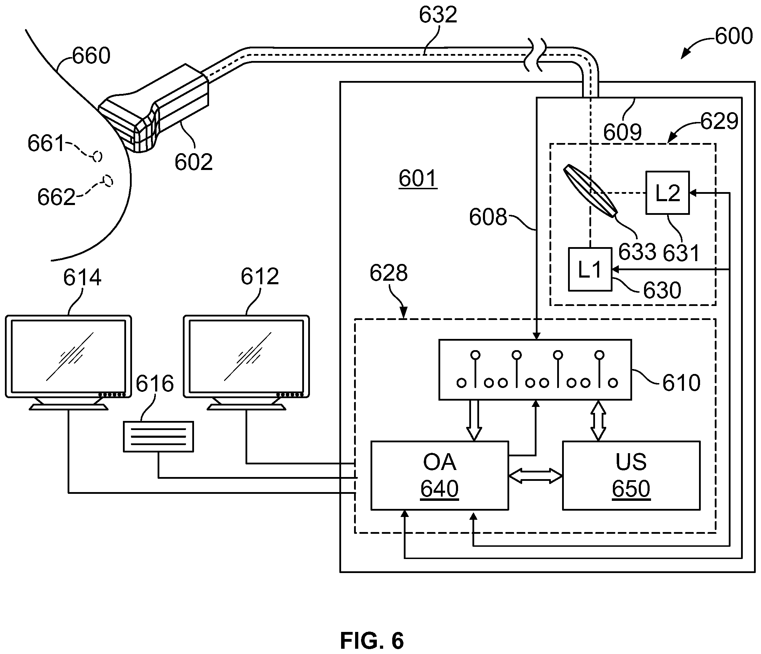

Figure 6 illustrates a block diagram of an optoacoustic system implemented in accordance with an embodiment herein. The optoacoustic system 600 includes a probe 602 connected via a light path 632 and an electrical path 608 to a system chassis 601. Within the system chassis 601 is housed a light subsystem 629 and a computing subsystem 628. The computing subsystem 628 includes one or more processors configured to implement specific program instructions for, among other things, optoacoustic control and analysis. In an embodiment, through the sampling of transducers in the probe 602, the optoacoustic system collects OA/US data in response to stimulation caused by pulsed light sources 630, 631 (i.e., the optoacoustic return signal) and to stimulation caused by acoustic output of the ultrasound transducer elements.

The optoacoustic system 600 comprises one or more light sources 630, 631 operating at different light wavelengths. In an embodiment, with light sources 630, 631 operating at different light wavelengths, the optoacoustic return signal from one light event from each of the light sources can be used in the method and system for presenting the optoacoustic data. In an embodiment, the system 600 comprises a single light source that may be operated at different wavelengths, such as a tunable laser that can change wavelengths quickly enough for use as described herein. In an embodiment, the system 600 comprises at least two light sources 630, 631, each being capable of tuning to a plurality of different wavelengths. In an embodiment, the system 600 comprises one light source 630 operating a one light wavelength, and at least one additional light source 631 capable of being tuned to a plurality of different wavelengths.

The one or more processors of the optoacoustic system 600 are configured to generate sinograms. The sinograms are processed to produce envelope images, including short envelope images and long envelope images. As used herein the term short envelope image refers to an envelope image corresponding to the short sinogram, and the term long envelope image refers to an envelope image corresponding to the long sinogram. In an embodiment, the short sinogram and long sinogram are each processed separately to produce a short envelope image and a long envelope image, respectively. The short and long envelope images are then used together to generate parametric images. From the parametric images, maps can be created of oxygenation, hemoglobin and masked oxygenation. These maps can be, in real time, co-registered data representing an ultrasound image of substantially the same volume, and can thereafter produce one or more of an oxygenation image, a hemoglobin image and a masked oxygenation image. In an embodiment, the oxygenation image, hemoglobin image and masked oxygenation image reflect information about the composition of the volume of tissue. The terms parametric map and parametric image are in some instances used interchangeably. The use of the term map generally relates to the correspondence between the image and a volume. Parametric maps may be represented in numerous ways, including, for example, as a single-channel (i.e., grayscale) representation, as a color (i.e., RGB) representation, or as a color with transparency (RGBA) representation. Parametric maps may be used to convey qualitative or quantitative information about one or more parameters. A parametric map or parametric image may be represented in computer memory or presented as a displayed representation, thus, as used herein, the term "image" or "map" do not necessarily imply a visual representation.

Sinograms may contain unwanted, inaccurate or insufficiently scaled data. These maladies of sinogram data may result from myriad reasons, including characteristics of the measuring instrument (e.g., the probe) or the light used, characteristics of the volume (i.e., the tissue), characteristics of the interaction between the volume and the probe or light, external stimuli, or other sources. Regardless of the source, a variety of processes can be used to remove unwanted aspects of the sinogram data.

Generally, in each of the following steps for processing the sinogram, the processing is performed on the time domain signal. In a preferred embodiment (and as discussed below) the probe 602 includes an acoustic lens that enables the sinogram data to be more focused on what is on the plane below that of the transducers - the image plane. In an illustrative embodiment, each channel of the sinogram data represents approximately 100 millimeters of distance in the volume. The acoustic lens generally rejects at least some portion of a signal propagating from points outside (e.g., orthogonal) to the image plane. Each transducer, however, receives signal from substantially all points of the image plane that lie within the approximately 100 millimeters distance.

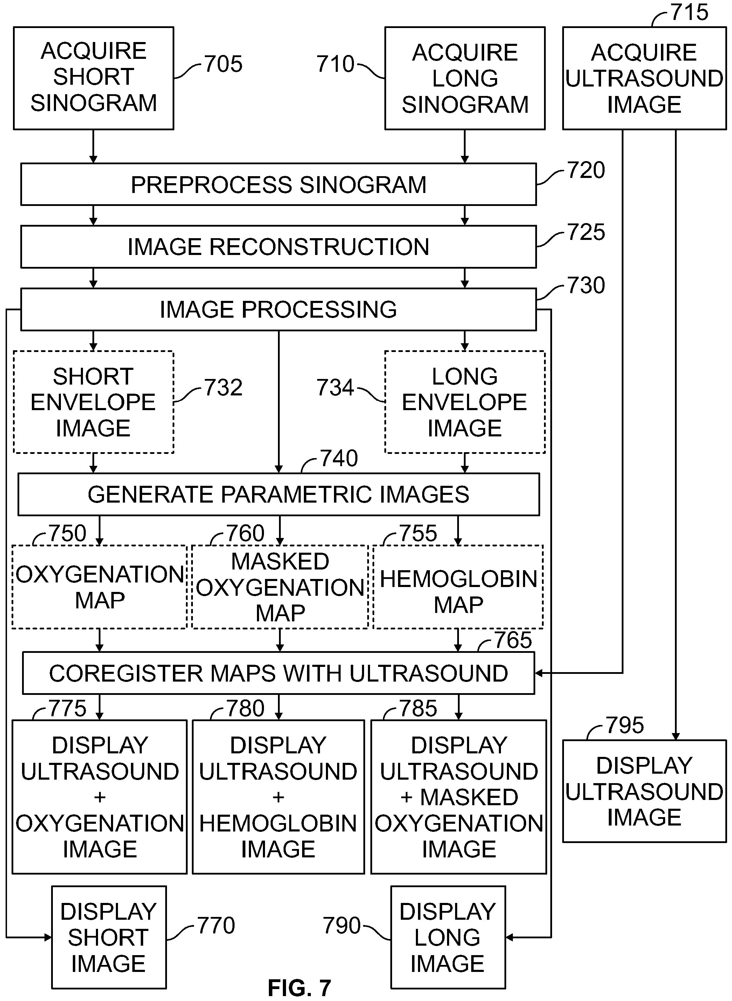

Figure 7 illustrates an overview of a collection of operations for processing OA/US data. Beginning with the acquisition of two sets of OA return signal data, namely, a short sinogram (step 705), a long sinogram (step 710), and acquisition of a US data set that is converted to an ultrasound image (step 715), the operations process the data to produce up to six separate images that may be useful in viewing various aspects of acquired OA/US data. For the purposes of illustration herein, it may be presumed that probe 602 movement is minimal, if any, between the acquisition of the three sets of data in steps 705, 710 and 715. It should be noted that the process described herein is not limited to being used with the three identified data sets. Use of additional data sets, such as, for example, data sets from additional wavelengths of light, may be used to further improve the resulting images. As will be discussed in more detail below, the short and long sinogram data are preprocessed (step 720) in one or more separate manners to reduce or compensate for undesired data in the sinogram, including characteristics of the measuring instrument (e.g., the probe) or the light used, characteristics of the volume (i.e., the tissue), characteristics of the interaction between the volume and the probe or light, external stimuli, or other sources. After the preprocessing, separate short and long images are reconstructed (step 725). In an embodiment, separate real and imaginary components of complex short and long images result from the reconstruction step.

In an embodiment, the processing (step 730) of the reconstructed images is performed. The operations described herein on connection with unmixing of module 108 (Figures 1-4) may be performed during the processing at step 730. The processing (step 730) may remove additional artifacts that can be identified in the reconstructed images, and in any event creates a short envelope image (732) and a long envelope image (734). In an embodiment, the short and long envelope images (732, 734) are used to generate parametric images (step 740). The parametric images (step 740) may include an oxygenation map (OA relative map) (750), a hemoglobin map (OA total hemoglobin map) (755) and a masked oxygenation map (OA combined map) (760). In an embodiment, any or all the three maps are co-registered with, and overlaid upon, a gray scale B-mode ultrasound image (step 765). A display can be provided for display of one or more of the displayable images displayed in steps 770, 775, 780, 785, 790 and 795. In an embodiment, a group of two or more of the images may be displayed on the same screen, and the displayed grouped images may be commonly scaled and sized. In an embodiment, the group of all six images may be displayed on the same screen, and it may be commonly scaled and sized.

In an embodiment, the system performing processing on the optoacoustic data, and/or the system displaying the optoacoustic output - which may but need not be the same as the system acquiring the sinogram -provides the operator the ability to vary parameters used in processing, when processing or viewing optoacoustic images. In an embodiment, the system performing processing on the optoacoustic data, and/or the system displaying the optoacoustic output would provide the operator the ability to switch on and off, and potentially vary the order of, the processing steps used to process the optoacoustic images.

The optoacoustic system 600 may also be employed as a multimodality, combined optoacoustic and ultrasound system. In an embodiment, the system 600 includes a probe 602 connected via a light path 632 and an electrical path 608 to a system chassis 601. Within the system chassis 601 is housed a light subsystem 629 and a computing subsystem 628. The computing subsystem 628 includes one or more computing components for ultrasound control and analysis and optoacoustic control and analysis; these components may be separate, or integrated. In an embodiment, the computing subsystem comprises a relay system 610, an optoacoustic processing and overlay system 640 and an ultrasound instrument 650. The system 600 may implement various types of network and data security measures, such as passwords, two-factor authentication, data encryption and the like.

In an illustrative embodiment, the light subsystem 629 may use Nd:YAG and Alexandrite lasers as its two light sources 630, 631, although other types or wavelengths, and additional lights, may also be used. Light sources 630, 631 should be capable of producing a short pulse of light, e.g., a pulse lasting less than about 100 ns, and more preferably around 5 ns. In an embodiment, the two light sources 630, 631 can be separately triggered. In an embodiment, the light output by the light sources 630, 631 may be projected onto the same light path 632 through the use of an optical element 633 that generally permits one light 630 to pass through from a first side to a second side, while reflecting one light source 631 that strikes the second side. The use of optical element 633 or a similar element permits the alignment of the output of two light sources 630, 631 such as lasers onto proximal end of the light path 632.

One or more displays 612, 614, which may be touch screen displays, are provided for displaying images and all or portions of the system user interface. One or more other user input devices 616 such as a keyboard, mouse and various other input devices (e.g., styluses, light pens, dials and switches) may be provided for receiving input from an operator. As an option, power and control path(s) 609 carry power to the probe 602 and control signals between the probe 602 and the computing subsystem 628.

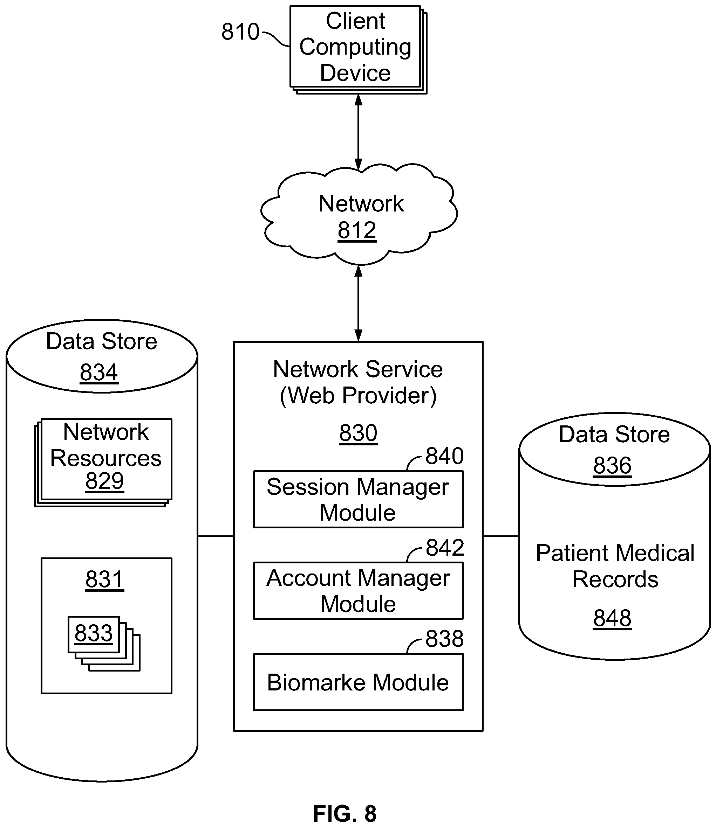

Figure 8 is a block diagram illustrating a web-based US/OA image management system formed in accordance with embodiments herein. The system includes one or more client computing devices 810 that communicate over a network 812, such as the Internet, with a web-based network service (e.g., web hosting provider) 830. The network service 830 may communicate with the client computing devices 810 over a common network or different networks. The client computing devices 810 may be implemented as any number of other types of computing devices. These devices may include, for instance, PCs, laptop computers, mobile phones, set-top boxes, game consoles, electronic book readers, a personal computer and a personal digital assistant (PDA) and so forth. The network 812 represents any one or combination of multiple different types of networks, such as cable networks, the Internet, private intranets, local area networks, wide area networks, wireless networks, and the like.

The network service 830 represents a site (e.g., a website) that is capable of handling requests from many users and serving, in response, various pages (e.g., web pages) that are rendered at the client computing devices 810. The network service 830 may, in response to client requests, perform the operations described herein in connection with Figures 1-4.

The site can be any type of medical network related site that supports user interaction, such as a medical network within a healthcare system, a privately managed medical record network and the like. The exchange of request and renderings between the client computing devices 810 and network service 830 will vary depending upon the level of applications and functionality performed locally on the client computing devices 810 and the level of applications and functionality performed at the network service 830. For example, in a network-central architecture, the network service 830 maintains the data and applications to perform substantially all of the substantive processing, such as assignment of non-Hb chromophore values, maintaining optical absorption coefficients of the non-Hb chromophores for different tissue types/subtypes, maintaining optical extinction coefficients and molar concentrations of the non-Hb chromophores for different tissue types/subtypes, a mass concentration and molecular weight of the non-Hb chromophores for different tissue types/subtypes, maintaining volume fractions of the non-Hb chromophores for different tissue types/subtypes. The network service 830 also collects and stores US/OA images, designations of interior and exterior outlines to be overlaid upon US/OA images, and the renderings of US/OA images with interior and exterior outlines thereon. The network service 830 may further render entry screens with entry fields associated with US/OA features of interest and convey the rendered US/OA images, entry fields and entry screens to the client computing devices 810. The client computing devices obtain feature scores for entry fields and return the feature scores to the network service 830. The network service 830 automatically enables and disables various entry fields to manage an order in which corresponding feature scores are entered such that the feature scores are obtained from at least one of the peripheral or boundary zones before feature scores are obtained from the internal zone of a corresponding one or more of the US/OA images.

Additionally, or alternatively, the system of Figure 8 may be implemented as a server - client distributed architecture, in which the network service 830 may provide applications for the client computing devices 810 to download, store, and run locally. For example, the applications may include a portion, or all the operations implemented in connection with Figures 1-4, as well as other processes described herein. In a distributed architecture, the network service 830 interacts with the client computing devices 810 to provide content as needed. For example, when a client computing device 810 does not already have US/OA images of interest, the client computing device 810 may request the US/OA images related to an examination or examinations for a particular patient. The client computing device 810 and/or the network service 830 may then designate the interior and exterior outlines to be overlaid upon US/OA images. For example, the interior and exterior outlines may be automatically generated based on image segmentation or otherwise. Additionally, or alternatively, one or both of the client computing device 810 and network service 830 may support a GUI that allows a user to draw and/or modify the interior and external outlines. The client computing device 810 and/or network service 830 may further render entry screens with entry fields associated with US/OA features of interest. The client computing devices 810 obtain feature scores for entry fields. The client computing device 810 and/or network service 830 automatically enable and disable various entry fields to manage an order in which corresponding feature scores are entered such that the feature scores are obtained from at least one of the peripheral or boundary zones before feature scores are obtained from the internal zone of a corresponding one or more of the US/OA images.

The network service 830 may represents a medical facility/network website that hosts patient medical records 848 in a data store 836 and an image catalog 831 that stores images 833. The data store 836 stores patient medical records, patient images, lookup tables and the like in connection with patient accounts. By way of example, the images 833 may represent image reference keys provided in connection with assisting readers and scoring features. Additionally, or alternatively, the images 833 may represent US images, OA images, mammogram images, and/or images from other modalities for the current patient, such as during prior examinations. Additionally, or alternatively, the images 833 may represent US images and/or OA images for other patients, that may be utilized by the reader to assist in classifying tissue types/subtypes and assigning feature scores. The patient medical record may include one or more of the following types of information (among other things): (a) patient medical history, (b) a history of examinations, medication, test results, (c) measurements taken in connection with a lesion, (d) mass diameter, (e) depth to posterior margin of the mass, (f) tissue types/subtypes, (g) chromophore factor values and the like. Additionally, or alternatively, the patient medical records may also include diagnostic imaging data collected from prior examinations, including but not limited to, ultrasound data sets, optoacoustic data sets, mammography data sets, MRI data sets, computed tomography sit data sets, positron emission tomography (PET) data sets, x-ray data sets, single photon emission computed tomography (SPECT) data sets, and the like. Sub-regions of images from the prior examinations may be classified with different tissue types/subtypes and/or assigned various chromophore factor values. The data store 836 may include, within or linked to the patient medical record, US images, OA images, BI-RADS ratings assigned in connection with reading mammography images and the like.

The network service 830 may also maintain authorization and licensing information in connection with client computing devices 810. For example, software applications, configured to implement portions or all the functionality described herein, may be purchased or licensed by users, with the applications downloaded to the client computing device 810. The network service 830 would then maintain any subscription related information concerning purchase, rental, licensing, viewing authorizations and the like in connection with each corresponding subscription.

The network service 830 includes, among other things, a session manager module 840, an account manager module 842 and a biomarker machine learning classifier (MLC) module 838. The modules 838, 840 and 842 cooperate, as described herein. The modules 838, 840 and 842 may be operated to improve technical efficiency, such as by providing offline or asynchronous data analytics on a per-account basis to provide recommendations and other features. The modules 838, 840 and 842, as well as other modules and services described herein, are implemented by one or more processors performing program instructions (stored in data store 834 or 836) to perform the operations described herein. The network service 830 interacts with one or more memories or data stores 834 and 836 in various manners as explained herein. One or both memories or data stores 834 and 836 may store program instructions to direct one or more processors to carry out the instructions described herein.

Further, the data store 834 may store network resources 829, such as files containing HTML code or other codes to define individual resources. A network resource 829 may correspond to a functionality provided in connection with embodiments herein. For example, a network resource 829 or a collection of network resources 829 may afford functionality to view various US images and select a US image of interest, view various OA images and select an OA image of interest, draw and/or modify interior and exterior outlines on the US and/or OA image, present entry screens for feature score entry fields, present pop-up windows with image reference keys and the like. As another example, one or a collection of network resources 829 may represent one or more classification models that are used to determine a predictive result based on a set of feature scores.

Network resources 829 are provided to the client computing device 810 in response to requests, such as one or more client requests. As one example, a network resource 829 may be defined by an index.html file that is saved at a particular HTTP address in the data store 834. Upon request from the client computing device 810, the network service 830 retrieves and provides a corresponding index.html file to the client computing device 810. As a client computing device 810 (e.g., a web browser operating on the client computing device 810) seeks to open and render web content component (e.g., HTTP elements) within the index.html file, the client computing device 810 encounters various HTML code strings/elements, for which additional request may be made of the network server 830 before a complete rendered webpage or other network resource may be presented. Among other things, a client request may include a query string seeking a response.

Definitions

The terms "BI-RADS" and "BR" shall mean Breast Imaging Reporting and Data System, and represents a method used by medical personnel to interpret and report in a standardized manner the results of mammography, ultrasound and MRI used in breast cancer screening and diagnosis. By way of example, a BI-RADS 3 score may be indicative of a 2% or less probability of malignancy, a BI-RADS 4A score may be indicative of a probability of malignancy between 2% and less than or equal to 10%, a lower BI-RADS 4B score may be indicative of a probability of malignancy of greater than 10% and less than or equal to 25% , an upper BI-RADS 4B score may be indicative of a probability of malignancy of greater than 25%, a lower BI-RADS 4C score may be indicative of a probability of malignancy of less than or equal to 75%, and upper BI-RADS 4C score may be indicative of a probability of malignancy of greater than 75%, and a BI-RADS 5 score may be indicative of a probability of malignancy of greater than or equal to 95%.

The term "biomarker" shall mean an objective medical sign that is a measurable and quantifiable indicator of a physiologic or pathologic state of a mass, defined structure and/or living organism. The term biomarker shall include a defined characteristic that is measured as an indicator of normal biologic processes, genetic processes, or responses to an exposure or intervention, including therapeutic interventions. A biomarker may be derived from any substance, structure or process that can be measured in the body or its products and influence or predict the incidence or outcome of disease (e.g., positive predictive value of breast cancer). A biomarker is not a "symptom" which is a subjective perception of health or illness. Different types of biomarkers exist. For example, a diagnostic biomarker may be used for the detection or confirmation of a disease or condition and for identification of a specific disease subtype (e.g., BI-RADS descriptors, ER, PR, and HER2 (also known as ERBB2) status). The diagnostic biomarker may represent a measurable and quantifiable indicator of whether to perform a biopsy or forgo a biopsy of a mass or other definable structure within a patient region of interest. As another example, a predictive biomarker may be used to identify individuals who are more likely to experience a favorable or unfavorable response to an intervention, medical product, or environmental exposure compared with individuals without the predictive biomarker. For example, the predictive biomarker may represent a possibility that mutations in BRCA genes are predictive of response to PARP inhibitors in patients with advanced breast and ovarian cancer. As another example, the predictive biomarker may represent the likelihood that ER- and PR-positive breast cancers respond to endocrine therapy. As another example, the predictive biomarker may represent a possibility that dense breast tissue is predictive of decreased sensitivity of mammography for detecting noncalcified breast cancer. As another example, a prognostic biomarker may represent a degree of angiogenesis and/or a likelihood of lymph node metastasis. A prognostic biomarker may indicate a percentage chance/probability of malignancy for a mass or other definable structure. As another example, a prognostic biomarker may reflect a likelihood of a clinical event, disease progression, or recurrence irrespective of an intervention (e.g., TNM stage, tumor grade, tumor receptor status). The prognostic biomarker may be an indicator of a molecular subtype for a malignancy.

The term "COI" shall refer to one or more chromophores of interest.

The term "diagnostic imaging data set" shall mean a data set acquired by one or more of an ultrasound system, optoacoustic system, computed tomography (CT) system, magnetic resonance imaging (MRI) system, positron emission tomography (PET) system, single-photon emission computed tomography (SPECT) system, x-ray system, angiography system, fluoroscopy system and the like. The data set may represent the raw data acquired by the corresponding system and/or one or more images generated from processing (e.g., rendering) the corresponding data set.

The terms "diagnostically relevant" and "diagnostic relevance" refers to an amount or value for a parameter, factor, chromophore and the like that will impact resulting images presented to a clinician (or computer based image analysis program) in an manner that may affect a diagnosis provided by the clinician or computer.

The term "extent", when describing a chromophore, refers to an amount, distribution, concentration of the chromophore(s).

The terms "feature" and "feature of interest" refer to features of an OA image, US image and feature combinations thereof. The non-OA features may be US features, MRI features, X-ray features, CT features, PET features, SPECT features or another medical diagnostic imaging modality. Nonlimiting examples of OA features include 1) internal vascularity and deoxygenation, 2) peri-tumoral boundary zone vascularity and deoxygenation, 3) internal deoxygenated blush, 4) internal total blood, 5) external peri-tumoral radiating vessels, and 6) interfering artifact. Non-limiting examples of ultrasound features include 1) US Shape Score, 2) US Internal Texture, 3) US Sound Transmission, 4) US Capsular or Boundary Zone, 5) US Peripheral Zone, 6) Patient Age, 7) Mammogram-BIRADS, 8) Lesion Size (cm), and 9) Lesion Posterior Depth (cm). Additional and alternative features are described in U.S. Patent 9,398,893, to Anthony Thomas Stavros et al., titled "system and method for diagnostic vector classification support", filed March 11, 2014 as application serial number 14/205, 005, and issuing July 26, 2016 (hereafter the Stavros '893 Patent), the complete and total subject matter of which is expressly incorporated herein by reference in its entirety.

The term "feature score" refers to a grade, rating, ranking or other evaluation information that is descriptive of one or more characteristics of a feature in an OA image and/or non-OA image. Non-limiting examples of feature scores include i) a numeric value along a range of numeric values, ii) a dimension measured from an OA or non-OA image, and/or iii) a word, phrase, or sentence describing a characteristic of the feature.

The term "functional information" refers to information in OA data indicative of physiological activity within a certain tissue or organ, including levels of oxygenate and de-oxygenated hemoglobin. Functional information is distinct from structural information.

The term "hematocrit" refers to the proportion of red blood cells in a given volume of blood.

The term "horizontal", when used to refer to a direction within a US or OA image, shall mean a direction perpendicular to a scanning direction of an ultrasound transmission/reception and/or optoacoustic transmission/reception.

The term "imaging biomarker" shall mean a biomarker that is present in, or derived from, an imaging data set and can be measured and quantified, from the imaging data set, to determine an indicator of a physiologic or pathologic state of the mass, defined structure and/or living organism within a region of interest, for which the imaging data set is obtained. An imaging biomarker represents a diagnostic, not a therapeutic, by providing useful information to guide therapy. An imaging biomarker may be semi-quantitative and may be an ordinal score in which the risk of a certain outcome increases with increasing ordinal score. Imaging procedures indirectly affect an outcome and utilize surrogate endpoints for accuracy (e.g., sensitivity, specificity, PPV, NPV, ROC, AUC). The endpoints are measurable and reproducible. Imaging biomarkers may not have a desired level of sensitivity or specificity to be utilized individually in isolation. However, select combinations of different imaging biomarkers will cumulatively provide a desired level of sensitivity and specificity. While embodiments herein describe certain combinations of different imaging biomarkers as applied utilizing ultrasound and/or Optoacoustic imaging of breast masses, it is recognized that the subject matter herein is not limited to the particular combinations of imaging biomarkers, nor the ultrasound and/or Optoacoustic imaging modalities, nor breast imaging. Instead, principals described herein may be applied to additional and alternative combinations of imaging biomarkers, additional and alternative imaging modalities, as well as other anatomical regions. Nonlimiting examples of descriptors for an imaging biomarker applicable to characterizing a mass as benign or malignant include margin, shape and orientation of the mass.

The term "LOM" shall mean likelihood or probability of malignancy.

The term "non-OA image" refers to any medical diagnostic image, other than an OA image, captured by one or more medical imaging modalities. A non-OA image constitutes an image that is captured based on an imaging principle that does not utilize transmission of optical light in two distinct frequency ranges to cause a volume of interest to generate acoustic signals. Non-limiting examples of non-OA images include ultrasound (US) images (transmissive and/or reflective), MRI images, X-ray images, CT images, PET images, and SPECT images. When the non-OA image is a US image, the US image may be captured by a US imaging system that is integrated with, coupled to or entirely separate from, an OA imaging system.

The term "NPV" shall mean negative predictive value.

The term "non" is used to express negation or absence of the subsequent element. For example, a "non-Hb chromophore" includes all chromophores but not hemoglobin chromophores. For example, a "non-collagen chromophore" includes all chromophores but not collagen chromophores.

The term "non-COI" shall refer to one or more chromophores not of interest.

The term "OA feature score" shall mean a feature score assigned based on one or more features of interest within an OA image.

The term "observation" refers to one or more OA images (alone or in combination with one or more non-OA images) that are collected from a patient during an OA examination. The observation may also include diagnostic information entered by a clinician, such as OA feature scores and/or non-OA feature scores.

The terms "optoacoustic image" and "OA image" refer to an image captured by an optoacoustic imaging system that utilizes transmit light at one or more frequencies into a volume of interest and receives an ultrasound data set that is processed and converted into an OA image.

The term "pathology data" shall include any and all test results from biopsies or other samples of a patient's tissue of interest and/or a patient's fluids (e.g., blood, urine, etc.). The term pathology data shall also include any clinician reports generated based on biopsies or other studies of the patient's tissue and/or fluids.

The term "predictive result" shall include qualitative diagnostic results, quantitative predictive results, prognostic results, monitoring results and the like. For the avoidance of doubt, a predictive result does not include or refer to a binary indication between whether to obtain a biopsy or not obtain a biopsy. The following represent a nonlimiting list of predictive results that may be obtained: i) percentage chance/probability of malignancy of a mass, ii) a likelihood of a clinical event, disease progression or reoccurrence, iii) molecular subtype, iv) histologic grade, v) degree of angiogenesis, vi) likelihood of lymph node metastasis, vii) assess the status of a disease or condition to find evidence of exposure to, or effects of, a medical product or environmental agent, viii) change in a patient condition (e.g., tumor size and volume), ix) indicator of an effect of a response to an exposure intervention, x) detect or confirm a disease or condition, xi) identify specific disease subtype, xii) possibility that mutations in genes are predictive of response to certain inhibitors, xiv) likelihood that ER and PR possible breast cancers respond to endocrine therapy, xv) possibility that dense breast tissue is predictive of decreased sensitivity of mammography for detecting noncalcified breast cancer, xvi) one of one or more molecular subtypes or xvii) histologic grades.

The term "PPV" shall mean positive predictive value, and the term "PPV3" shall mean biopsy proven positive predictive value.

The terms "obtain" or "obtaining", as used in connection with data, signals, information and the like, includes at least one of i) accessing memory of various systems or devices (e.g., a diagnostic imaging system, PACS workstation, medical network workstation, desktop computer, laptop computer, tablet device, smart phone or remote server) where the data, signals, information, etc. are stored, ii) receiving the data, signals, information, etc. over a wireless communications link between the various systems and devices, and/or iii) receiving the data, signals, information, etc. at a remote server over a network connection. The obtaining operation, when from the perspective of a diagnostic imaging system, may include collecting new imaging data in real time, and/or accessing memory to read stored data, signals, information, etc. from memory within the diagnostic imaging system. The obtaining operation, when from the perspective of a local non-imaging device (e.g., PACS workstation, medical network workstation, desktop computer, laptop computer, tablet device, smart phone), includes receiving the data, signals, information, etc. at a transceiver of the local non-imaging device where the data, signals, information, etc. are transmitted from the diagnostic imaging system and/or a remote server. The obtaining operation may be from the perspective of a remote server, such as when receiving the data, signals, information, etc. at a network interface from a local non-imaging device and/or directly from a diagnostic imaging system. The remote server may also obtain the data, signals, information, etc. from local memory and/or from other memory, such as within a cloud storage environment and/or from the memory of a workstation or clinician external programmer.

The terms "receive" and "receiving" when used in connection with OA/US feature scores and/or OA/US images, includes at least one of i) collecting OA/US data sets in real time from a diagnostic imaging system, while performing a patient scan; ii) receiving inputs, such as OA/US feature scores entered by medical personnel; iii) receiving an automatic output of a machine learning classifier that automatically assigns OA/US feature scores; iv) receiving the data, signals, information, etc. over a wired or wireless communications link between the various systems and devices, and/or iii) receiving the data, signals, information, etc. at a remote server over a network connection.

The term "UL feature score" shall mean a feature score assigned based on one or more features of interest within an ultrasound only image.

The terms "US" and "UL" are used interchangeably to refer to ultrasound only, and not opto-acoustics.

The terms "OA/US", "OA/UL", "OA/US" and "UL/OA" shall mean ultrasound and/or optoacoustic and shall include ultrasound only, optoacoustic only, or a combination of ultrasound and optoacoustic. For example, a OA/US data set (or US/OA data set) may include 1) only a US data set, with no OA data, 2) an OA data set, with no US data, or e) a US data set and an OA data set. As another example, a OA/US feature score (or US/OA feature score) may include 1) only a US feature score, with no OA feature score, 2) an OA feature score, with no US feature score, or 3) a US feature score and an OA feature score. As another example, an OA/US image (or US/OA image) may include 1) only a US image, with no OA image, 2) an OA image, with no US image, or 3) a US image and an OA image.

The term "sinogram" refers to an OA data set of acoustic activity occurring over a period of time in response to one or more light events impinging on the tissue site. The acoustic activity captured in the sinogram include an optoacoustic response, i.e., the acoustic signal that is created as a result of the electromagnetic energy being absorbed by materials within the tissue site such as, for example, various tissue structures that absorb the electromagnetic energy. These acoustic signals result from the release of thermo-elastic stress confinement within the tissue structures in response to the light events.

The term "vertical", when used to refer to a direction within a US or OA image, shall mean a direction parallel to a scanning direction of an ultrasound transmission/reception and/or opto-acoustic transmission/reception.

The term "volume fraction" refers to the volume of a constituent divided by the volume of all constituents of the mixture. The volume fraction coincides with the volume concentration in ideal solutions where the volumes of the constituents are additive (the volume of the solution is equal to the sum of the volumes of its ingredients).

Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices and program products according to various example embodiments. These program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications/controllers/computing components herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally, or alternatively, the units/modules/applications/controllers/computing components herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications/controllers/computing components herein may execute a set of instructions that are stored in one or more storage elements, in order to process data. The storage elements may also store data or other information as desired or needed. The storage element may be in the form of an information source or a physical memory element within the units/modules/applications/controllers/computing components herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of obj ect-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

**Claims**

1. An optoacoustic (OA) imaging system, comprising:

   memory configured to store program instructions; and
   one or more processors configured to execute the programmable instructions to:

   obtain OA return signal data associated with a response of a sub-region of a region of interest (ROI) to one or more first laser light pulses having a first predominant wavelength;
   generate an acoustic pressure data set based on the OA return signal data, the acoustic pressure data associated with the sub-region of the ROI, wherein the acoustic pressure data is dependent on a composition of hemoglobin (Hb) and non-Hb chromophores in the sub-region;
   identify an extent of the at least one non-Hb chromophore within the sub-region;
   assign a value to a non-Hb chromophore factor based on the extent of the at least one non-Hb chromophore within the sub-region; and
   compute an amount of at least one of i) the Hb chromophore or ii) a second non-Hb chromophore in the sub-region based on the acoustic pressure data and the value assigned to the non-Hb chromophore factor.

2. The system of claim 1, wherein the one or more processors are further configured to identify the extent of at least one non-Hb chromophore within the sub-region, by at least one of:

   i) analyzing an imaging data set for the sub-region to determine an aspect of the composition related to the extent of the one non-Hb chromophore; or
   ii) analyzing pathology data indicative of the extent of the non-Hb chromophore; or
   iii) receiving patient data indicative of the extent of the non-Hb chromophore.

3. The system of claims 1 or 2, wherein the ROI includes first and second sub-regions having different first and second compositions, the one or more processors further configured to identify first and second extents of the non-Hb chromophore within the first and second sub-regions, respectively, and, based thereon, to assign first and second values to the non-Hb chromophore factor for the first and second sub-regions, respectively.

4. The system of any one of claims 1-3, wherein, to identify the extent, the one or more processors are further configured to, at least one of:

   i) determine a volume fraction of the non-Hb chromophore in the sub-region,
   ii) classify the sub-region into one or more tissue types, or
   iii) determine a concentration of the non-Hb chromophore in the sub-region.

5. The system of any one of claims 1-4, wherein the acoustic pressure data for the ROI represents an OA image and wherein the one or more processors are further configured to:

   apply a fluence adjustment to the acoustic pressure data of the OA image to form a fluence-adjusted OA image; and
   compute a parametric map based on the fluence-adjusted OA image after applying the fluence adjustment.

6. The system of any one of claims 1-5, wherein the one or more processors are further configured to assign the value for the non-Hb chromophore factor based on at least one of: i) an optical absorption coefficient of the non-Hb chromophore in the sub-region, ii) an optical extinction coefficient and molar concentration of the non-Hb chromophore in the sub-region, iii) a mass concentration and molecular weight of the non-Hb chromophore in the sub-region, or iv) a volume fraction of the non-Hb chromophore in the sub-region.

7. The system of any one of claims 1-6, wherein the non-Hb chromophore factor corresponds to at least one of: i) an optical absorption coefficient of the non-Hb chromophore in the sub-region, ii) an optical extinction coefficient of the non-Hb chromophore in the sub-region, iii) a molar concentration of the non-Hb chromophore in the sub-region, iv) a mass concentration of the non-Hb chromophore in the sub-region, or iv) a volume fraction of the non-Hb chromophore in the sub-region.

8. The system of any one of claims 1-7, wherein the non-Hb chromophore factor corresponds to a non-blood absorption coefficient and wherein, to compute the amount of the Hb chromophore, the one or more processors are further configured to:

determine a tissue absorption coefficient based on the acoustic pressure data;
determine a blood absorption coefficient based on the tissue absorption coefficient and the value of the non-blood absorption coefficient; and
determine the amount of the Hb chromophore based on the blood absorption coefficient and an Hb extinction coefficient.

9. The system of any one of claims 1-8, wherein the non-Hb chromophore includes at least one of a water chromophore, melanin chromophore or lipid chromophore, or wherein the Hb chromophore includes at least one of an oxygenated hemoglobin (HbO) chromophore or a deoxygenated hemoglobin (HbR) chromophore.

10. The system of any one of claims 1-9, further comprising:

one or more light sources configured to generate a first laser light pulse having a first predominant wavelength;
an OA probe operatively coupled to the one or more light sources, the OA probe configured to deliver the first laser light pulse to a region of interest (ROI) of tissue, the ROI including i) at least one non-hemoglobin (non-Hb) chromophore, and ii) at least one hemoglobin (Hb) chromophore; and
the OA probe including a transducer array that is configured to collect OA return signal data associated with a response of the ROI to one or more of the first laser light pulses.

11. The system of any one of claims 1-10, further comprising a network service housing the memory and the one or more processors, the network service configured to receive, over a network connection, at least one of the OA return signal data or acoustic pressure data from one or more OA imaging systems.

12. An optoacoustic (OA) imaging method, comprising:
utilizing one or more processors configured to execute the programmable instructions for:

obtaining OA return signal data associated with a response of a region of interest (ROI) to one or more of first laser light pulses;generating an acoustic pressure data set based on the OA return signal data, the acoustic pressure data associated with the sub-region of the ROI, wherein the acoustic pressure data is dependent on a composition of hemoglobin (Hb) and non-Hb chromophores in the sub-region;
identifying an extent of the at least one non-Hb chromophore within the sub-region;
assigning a value to a non-Hb chromophore factor based on the extent of the at least one non-Hb chromophore within the sub-region; and
computing an amount of at least one of i) the Hb chromophore or ii) a second non-Hb chromophore in the sub-region based on the acoustic pressure data and the value assigned to the non-Hb chromophore factor.

13. The method of claim 12, wherein the identifying the extent of the at least one non-Hb chromophore within the sub-region, includes at least one:

i) analyzing an imaging data set for the sub-region to determine an aspect of the composition related to the extent of the one non-Hb chromophore; or
ii) analyzing pathology data indicative of the extent of the non-Hb chromophore; or
iii) receiving patient data indicative of the extent of the non-Hb chromophore.

14. The method of claims 12 or 13, wherein the ROI includes first and second sub-regions having different first and second compositions, the method comprising identifying first and second extents of the non-Hb chromophore within the first and second sub-regions, respectively, and, based thereon, assigning first and second values to the non-Hb chromophore factor for the first and second sub-regions, respectively.

**15.** The method of any one of claims 12-14, wherein, the identifying the extent, includes at least one of:

> i) determining a volume fraction of the non-Hb chromophore in the sub-region,
> ii) classifying the sub-region into one or more tissue types, or
> iii) determining a concentration of the non-Hb chromophore in the sub-region.

**16.** The method of any one of claims 12-15, wherein the acoustic pressure data for the ROI represents an OA image and wherein the method further comprises:

> applying a fluence adjustment to the acoustic pressure data of the OA image to form a fluence-adjusted OA image; and
> computing a parametric map based on the fluence-adjusted OA image after applying the fluence adjustment.

**17.** The method of any one of claims 12-16, further comprising assigning the value for the non-Hb chromophore factor based on at least one of:

> i) an optical absorption coefficient of the non-Hb chromophore in the sub-region,
> ii) an optical extinction coefficient and molar concentration of the non-Hb chromophore in the sub-region,
> iii) a mass concentration and molecular weight of the non-Hb chromophore in the sub-region, or
> iv) a volume fraction of the non-Hb chromophore in the sub-region.

**18.** The method of any one of claims 12-17, wherein the non-Hb chromophore factor corresponds to at least one of:

> i) an optical absorption coefficient of the non-Hb chromophore in the sub-region,
> ii) an optical extinction coefficient of the non-Hb chromophore in the sub-region,
> iii) a molar concentration of the non-Hb chromophore in the sub-region,
> iv) a mass concentration of the non-Hb chromophore in the sub-region,
> v) a volume fraction of the non-Hb chromophore in the sub-region; or
> vi) a non-blood absorption coefficient and the computing the amount of the Hb chromophore includes: determining a tissue absorption coefficient based on the acoustic pressure data; determining a blood absorption coefficient based on the tissue absorption coefficient and the value of the non-blood absorption coefficient; and determining the amount of the Hb chromophore based on the blood absorption coefficient and an Hb extinction coefficient.

**19.** The method of any one of claims 12-18, further comprising: delivering a first laser light pulse, having a first predominant wavelength, a region of interest (ROI) of tissue, the ROI including i) at least one non-hemoglobin (non-Hb) chromophore, and ii) at least one hemoglobin (Hb) chromophore.

**20.** The method of any one of claims 12-19, further comprising receiving, at a network service housing the memory and the one or more processors, at least one of the OA return signal data or acoustic pressure data from one or more OA imaging systems.

**21.** The method of any one of claims 12-20, wherein the first and second chromophores represent one of the following:

> a) the first chromophore represents a hemoglobin (Hb) chromophore and the one or more second chromophores represent one or more non-Hb chromophores;
> b) the first chromophore represents a collagen chromophore and the one or more second chromophores represent one or more non-collagen chromophores; or
> c) the first chromophore represents a lipid chromophore and the one or more second chromophores represent one or more non-lipid chromophores.

**FIG. 1**

EP 4 434 447 A1

**FIG. 2**

Source (chromophone) unmixing

FIG. 3

**FIG. 4**

502

Obtain Patient data, Pathology Data and/or Images
(Real-time or prior)

504

Segment ROI into one or more sub-regions

506

Classify tissue type or subtype(s) in ROI
and/or sub-regions

508

Determine one or more values for one or more
Hb and/or non-Hb Chromaphone factors

510

Store value(s) (e.g, in look up table) or assign
value(s) to current factors (e.g, for real time use)

**FIG. 5A**

```
                                                    ┌─522
┌──────────────────────────────────────────────────┐
│        Obtain Patient data, Pathology Data         │
│              and/or Image Data                     │
└──────────────────────────────────────────────────┘
                        │
                        ▼                           ┌─524
┌──────────────────────────────────────────────────┐
│        Segment ROI into one or more sub-regions    │
└──────────────────────────────────────────────────┘
                        │
                        ▼                           ┌─526
┌──────────────────────────────────────────────────┐
│        Classify tissue type or subtype(s) in ROI   │
│                  and/or sub-regions                │
└──────────────────────────────────────────────────┘
                        │
                        ▼                           ┌─528
┌──────────────────────────────────────────────────┐
│      Determine one or more values for one or more  │
│      collagen and/or non-collagen Chromaphones     │
└──────────────────────────────────────────────────┘
                        │
                        ▼                           ┌─530
┌──────────────────────────────────────────────────┐
│              Store value(s) or assign              │
│             values to current factors              │
└──────────────────────────────────────────────────┘
```

**FIG. 5B**

**FIG. 6**

**FIG. 7**

810 — Client Computing Device

Network 812

Data Store 834

Network Resources 829

831

833

Network Service (Web Provider) 830

840

Session Manager Module

842

Account Manager Module

838

Biomarke Module

Data Store 836

Patient Medical Records 848

FIG. 8

Europäisches Patentamt

European Patent Office

Office européen des brevets

# EUROPEAN SEARCH REPORT

**Application Number**

EP 24 16 6071

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/266725 A1 (ZALEV JASON [CA] ET AL) 29 August 2019 (2019-08-29) * paragraphs [0241], [0296], [0339], [0379] - [0380]; figure 1 * ----- | 1-21 | INV. A61B5/00 |
| X | US 2023/026419 A1 (GRASSO VALERIA [NL] ET AL) 26 January 2023 (2023-01-26) * paragraphs [0031] - [0033]; claim 11 * * the whole document * ----- | 1-21 | |

**TECHNICAL FIELDS SEARCHED (IPC)**

A61B

The present search report has been drawn up for all claims

| Place of search | Date of completion of the search | Examiner |
|---|---|---|
| The Hague | 6 August 2024 | Furlan, Stéphane |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...................................................................................................
& : member of the same patent family, corresponding
    document

EPO FORM 1503 03.82 (P04C01)

**EP 4 434 447 A1**

## ANNEX TO THE EUROPEAN SEARCH REPORT
## ON EUROPEAN PATENT APPLICATION NO.

EP 24 16 6071

06-08-2024

| Patent document cited in search report | | Publication date | Patent family member(s) | | Publication date |
|---|---|---|---|---|---|
| US 2019266725 | A1 | 29-08-2019 | NONE | | |
| US 2023026419 | A1 | 26-01-2023 | CA | 3167234 A1 | 07-10-2021 |
| | | | CN | 115209795 A | 18-10-2022 |
| | | | EP | 4125558 A1 | 08-02-2023 |
| | | | JP | 2023520007 A | 15-05-2023 |
| | | | US | 2023026419 A1 | 26-01-2023 |
| | | | WO | 2021202438 A1 | 07-10-2021 |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63491745
- US 20220370037 A1
- EP 2208465 A1
- US 20220240890 A1
- US 20140288425 A1
- US 20170090571 A1
- WO 2018046095 A1
- US 20220225967 A1
- EP 3740132 A1
- US 20210113190 A1
- WO 2021069216 A1
- WO 2022069377 A1
- US 11452503 B2
- US 10338203 B2
- US 9465090 B2
- FR 2934054 B1
- EP 3787519 B1
- US 9398893 B, Anthony Thomas Stavros
- US 14205005 B

**Non-patent literature cited in the description**

- **RADHAKRISHNA et al.** Role of magnetic resonance imaging in breast cancer management. *South Asian J Cancer,* 2018, vol. 7, 69-71
- **HYSI, ENO et al.** Photoacoustic imaging of kidney fibrosis for assessing pretransplant organ quality. *JCI insight,* 2020, vol. 5, 10